# EUROPEAN PATENT SPECIFICATION

(11) **EP 1 213 965 B1**
(45) Date of publication and mention of the grant of the patent: **18.01.2006**
(21) Application number: 00961674.9
(22) Date of filing: 08.09.2000
(51) Int. Cl.: A61K 31/415, A61P 7/00, A61P 7/04, A61P 7/06, C07D 231/22, C07D 231/26

(54) **THROMBOPOIETIN MIMETICS**
THROMBOPOIETIN-MIMETIKA
MIMETIQUES DE THROMBOPOIETINE

(30) Priority: 10.09.1999 US 153358 P; 12.05.2000 US 203668 P
(43) Date of publication of application: 19.06.2002
(73) Proprietor: SMITHKLINE BEECHAM CORPORATION, Philadelphia, Pennsylvania 19101 (US)
(72) Inventor: LUENGO, Juan, I., Audubon, PA 19403 (US); DUFFY, Kevin, J., Norristown, PA 19403 (US)
(74) Representative: Breen, Anthony Paul
(86) International application number: PCT/US2000/024665
(87) International publication number: WO 2001/017349

(56) References cited:
- WO-A-00/35446
- US-A- 5 482 546
- KIMURA T ET AL: "A non-peptide compound which can mimic the effect of thrombopoietin via c-Mpl" FEBS LETTERS, ELSEVIER SCIENCE PUBLISHERS, AMSTERDAM, NL, vol. 428, no. 3, 29 May 1998 (1998-05-29), pages 250-254, XP004257949 ISSN: 0014-5793
- DUFFY K J ET AL: "HYDRAZINONAPHTHALENE AND AZONAPHTHALENE THROMBOPOIETIN MIMICS ARE NONPEPTIDYL PROMTERS OF MEGAKARYOCYTOPOIESIS" JOURNAL OF MEDICINAL CHEMISTRY, AMERICAN CHEMICAL SOCIETY. WASHINGTON, US, vol. 44, no. 22, 25 October 2001 (2001-10-25), pages 3730-3745, XP001068980 ISSN: 0022-2623

## Description

### FIELD OF THE INVENTION

This invention relates to thrombopoietin (TPO) mimetics and their use as promoters of thrombopoiesis and megakaryocytopoiesis.

### BACKGROUND OF THE INVENTION

Megakaryocytes are bone marrow-derived cells, which are responsible for producing circulating blood platelets. Although comprising <0.25% of the bone marrow cells in most species, they have >10 times the volume of typical marrow cells. See Kuter et al. Proc. Natl. Acad. Aci. USA 91: 11104-11108 (1994). Megakaryocytes undergo a process known as endomitosis whereby they replicate their nuclei but fail to undergo cell division and thereby give rise to polypoid cells. In response to a decreased platelet count, the endomitotic rate increases, higher ploidy megakaryocytes are formed, and the number of megakaryocytes may increase up to 3-fold. See Harker J. Clin. Invest. 47: 458-465 (1968). In contrast, in response to an elevated platelet count, the endomitotic rate decreases, lower ploidy megakaryocytes are formed, and the number of megakaryocytes may decrease by 50%.

The exact physiological feedback mechanism by which the mass of circulating platelets regulates the endomitrotic rate and number of bone marrow megakaryocytes is not known. The circulating thrombopoietic factor involved in mediating this feedback loop is now thought to be thrombopoietin (TPO). More specifically, TPO has been shown to be the main humoral regulator in situations involving thrombocytopenia. See, e.g., Metcalf Nature 369:519-520 (1994). TPO has been shown in several studies to increase platelet counts, increase platelet size, and increase isotope incorporation into platelets of recipient animals. Specifically, TPO is thought to affect megakaryocytopoiesis in several ways: (1) it produces increases in megakaryocyte size and number, (2) it produces an increase in DNA content, in the form of polyploidy, in megakaryocytes; (3) it increases megakaryocyte endomitosis; (4) it produces increased maturation of megakaryocytes; and (5) it produces an increase in the percentage of precursor cells, in the form of small acetylcholinesterase-positive cells, in the bone marrow.

Because platelets (thrombocytes) are necessary for blood clotting and when their numbers are very low a patient is at risk of death from catastrophic hemorrhage, TPO has potential useful application in both the diagnosis and the treatment of various hematological disorders, for example, diseases primarily due to platelet defects. Ongoing clinical trials with TPO have indicated that TPO can be administered safely to patients. In addition, recent studies have provided a basis for the projection of efficacy of TPO therapy in the treatment of thrombocytopenia, and particularly thrombocytopenia resulting from chemotherapy, radiation therapy, or bone marrow transplantation as treatment for cancer or lymphoma. See e.g., McDonald (1992) Am. J. Ped. Hematology/Oncology 14: 8-21 (1992).

The gene encoding TPO has been cloned and characterized. See Kuter et al., Proc. Natl. Acad. Sci. USA 91: 11104-11108 (1994); Barley et al., Cell 77: 1117-1124 (1994); Kaushansky et al., Nature 369:568-571 (1994); Wendling et al., Nature 369: 571-574 (1994); and Sauvage et al., Nature 369: 533-538 (1994). Thrombopoietin is a glycoprotein with at least two forms, with apparent molecular masses of 25 kDa and 31 kDa, with a common N-terminal amino acid sequence. See, Bartley, et al., Cell 77: 1117-1124 (1994). Thrombopoietin appears to have two distinct regions separated by a potential Arg-Arg cleavage site. The amino-terminal region is highly conserved in man and mouse, and has some homology with erythropoietin and interferon-a and interferon-b. The carboxy-terminal region shows wide species divergence.

The DNA sequences and encoded peptide sequences for human TPO receptor (TPO-R; also known as c-mpl) have been described. See, Vigon et al. Proc. Natl. Acad. Sci. USA 89: 5640-5644 (1992). TPO-R is a member of the haematopoietin growth factor receptor family, a family characterized by a common structural design of the extracellular domain, including for conserved C residues in the N-terminal portion and a WSXWS motif close to the transmembrane region. See Bazan Proc. Natl. Acad. Sci. USA 87: 6934-6938 (1990). Evidence that this receptor plays a functional role in hematopoiesis includes observations that its expression if restricted to spleen, bone marrow, or fetal liver in mice (see Souyri et al. Cell 63: 1137-1147 (1990)) and to megakaryocytes, platelets, and CD34⁺ cells in humans (see Methia et al. Blood 82: 1395-1401 (1993)). Further evidence for TPO-R as a key regulator of megakaryopoiesis is the fact that exposure of CD34⁺ cells to synthetic oligonucleotides antisense to TPO-R RNA significantly inhibits the appearance of megakaryocyte colonies without affecting erythroid or myeloid colony formation. Some workers postulate that the receptor functions as a homodimer, similar to the situation with the receptors for G-CSF and erythropoietin.

The slow recovery of platelet levels in patients suffering from thrombocytopenia is a serious problem, and has lent urgency to the search for a blood growth factor agonist able to accelerate platelet regeneration.

It would be desirable to provide compounds which allow for the treatment of thrombocytopenia by acting as a TPO mimetic.

WO 00/35446 Al, published on 22 June 2000, discloses *inter alia* 3-hydroxy-4-[substituted 5-hydroxy-1H-pyrazol-4-yl]azo-1-naphthalenesulfonic acids and naphthalenecarboxylic acids as TPO mimetics, and their use in treating thrombocytopenia. US 5,482,546 discloses substituted [5-hydroxy-1H-pyrazol-4-yl]-azo-benzene compounds as dyes and inks suitable for ink-jet uses. T. Kimura et al., *FEBS Lett.,* 1998, 428, 250-254 discloses a benzodiazepinone which mimics the effect of thrombopoietin via c-Mpl.

As disclosed herein it has unexpectedly been discovered that certain hydroxy-1-azobenzene derivatives are effective as agonists of the TPO receptor, they are potent TPO mimetics.

### SUMMARY OF THE INVENTION

This invention relates to a medicament for use in therapy, manufactured using and comprising a compound of Formula (I): 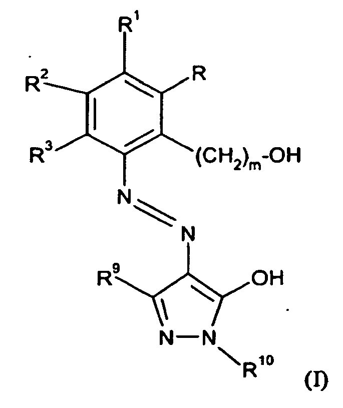
wherein:
R, R¹, R², R³ and R⁹ are each independently selected from hydrogen, C₁₋₆alkyl, C₁₋₆alkoxy, -(CH₂)ₚOR⁴, -C(O)OR⁴, nitro, cyano, halogen, aryl, -S(O)ₙR⁴, cycloalkyl, -CONR⁵R⁶, phosphonic acid, sulfonic acid, phosphinic acid and -SO₂NR⁵R⁶,
   where
   p is 0-6;
   n is 0-2;
   R⁴ is hydrogen, alkyl, cycloalkyl, C₁-C₁₂aryl, substituted alkyl, substituted cycloalkyl and substituted C₁-C₁₂aryl, and
   R⁵ and R⁶ are each independently selected from hydrogen, alkyl, C₃₋₆cycloalkyl, and aryl; or R⁵ and R⁶ taken together with the nitrogen to which they are attached represent a 5 to 6 member saturated ring containing up to one other heteroatom selected from oxygen and nitrogen;
m is 0-6; and
R¹⁰ is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, cycloalkyl, substituted cycloalkyl, aryl, substituted aryl, aryloxy, alkoxy, acyloxy, amino, nitro, cyano, halogen, hydroxy, and alkyl substituted with one or more substituents selected from the group consisting of: alkoxy, acyloxy, aryl, substituted aryl, cycloalkyl, substituted cycloalkyl, aryloxy, amino, nitro, cyano, halogen, and hydroxy; and pharmaceutically acceptable salts, hydrates, solvates and esters thereof;
provided that:
at least one of R, R¹, R² and R³ is: sulfonic acid, -C(O)OR⁴, tetrazole, -CONR⁵R⁶, phosphonic acid or phosphinic acid; where R⁴, R⁵ and R⁶ are as described above;
and provided that:
when R¹ is carboxylic acid; R, R² and R³ are hydrogen; and R⁹ is methyl;
R¹⁰ is not unsubstituted phenyl.

This invention also relates to the use of a compound of Formula (II): 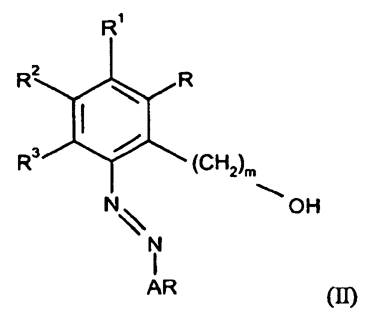
wherein:
R, R¹, R² and R³ are each independently selected from hydrogen, C₁₋₆alkyl, C₁₋₆alkoxy, -(CH₂)ₚOR⁴, -C(O)OR⁴, nitro, cyano, halogen, aryl, -S(O)ₙR⁴, cycloalkyl, -CONR⁵R⁶, -NR⁵R⁶, phosphonic acid, sulfonic acid, phosphinic acid and -SO₂NR⁵R⁶,
   where
   p is 0-6;
   n is 0-2;
   R⁴ is hydrogen, alkyl, cycloalkyl, C₁-C₁₂aryl, substituted alkyl, substituted cycloalkyl and substituted C₁-C₁₂aryl and
   R⁵ and R⁶ are each independently selected from hydrogen, alkyl, C₃₋₆cycloalkyl, and aryl,
   or R⁵ and R⁶ taken together with the nitrogen to which they are attached represent a 5 to 6 member saturated ring containing up to one other heteroatom selected from oxygen and nitrogen;
m is 0-6; and
AR is cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, aryl, substituted cycloalkyl, substituted aryl, aryloxy, hydroxy, alkoxy, cycloalkyl, acyloxy, amino, N-acylamino, nitro, cyano, halogen, -C(O)OR⁴, -C(O)NR⁷R⁸, -S(O)₂R⁷R⁸, -S(O)ₙR⁴, and alkyl substituted with one or more substituents selected from the group consisting of: alkoxy, acyloxy, aryl, substituted aryl, amino, N-acylamino, oxo, hydroxy, cycloalkyl, substituted cycloalkyl, -C(O)OR⁴, -C(O)NR⁷R⁸, -S(O)₂NR⁷R⁸, -S(O)ₙR⁴, aryloxy, nitro, cyano, and halogen,
   where
   R⁴ is hydrogen, alkyl, cycloalkyl, C₁-C₁₂aryl, substituted alkyl, substituted cycloalkyl and substituted C₁-C₁₂aryl; and
   R⁷ and R⁸ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴, -S(O)ₙR⁴, -C(O)NR⁴R⁴, -S(O)₂NR⁴R⁴, nitro, cyano, cycloalkyl, substituted cycloalkyl, halogen, C₁-C₁₂aryl, and substituted C₁-C₁₂aryl,
   or R⁷ and R⁸ taken together with the nitrogen to which they are attached represent a 5 to 6 member saturated ring containing up to one other heteroatom selected from oxygen and nitrogen,
   where R⁴ is as described above and n is 0-2; and
   pharmaceutically acceptable salts, hydrates, solvates and esters thereof;
in the manufacture of a medicament for use in treating thrombocytopenia.

The present invention also relates to the discovery that the compounds of Formula (II) are active as agonists of the TPO receptor.

In a further aspect of the disclosure there is provided novel processes and novel intermediates useful in preparing the presently invented TPO mimetic compounds.

Included in the present invention are pharmaceutical compositions comprising a pharmaceutical carrier and compounds useful in the methods of the invention.

Also useful are methods of co-administering the presently invented TPO mimetic compounds with further active ingredients.

### DETAILED DESCRIPTION OF THE INVENTION

The present compounds that act as TPO mimetics are defined by Formula (I) above.

Preferred among the present Formula I compounds are those in which R¹ is carboxylic acid or sulfonic acid; R, R² and R³ are each independently selected from hydrogen, carboxylic acid, C₁-C₁₂aryl, sulfonic acid, tetrazole, -CONR⁵R⁶ where R⁵ and R⁶ are as described in Formula I above, phosphonic acid, phosphinic acid, C₁₋₆alkoxy, nitro, C₁₋₆alkyl and halogen; m is 0; R⁹ is C₁₋₆alkyl, C₁₋₆alkoxy, halogen, or C₁-C₁₂aryl; and R¹⁰ is a cyclic or polycyclic aromatic ring containing from 3 to 14 carbon atoms, optionally containing one or more heteroatoms, provided that when the number of carbon atoms is 3 the aromatic ring contains at least two heteroatoms and when the number of carbon atoms is 4 the aromatic ring at least one heteroatom, and optionally substituted with one or more substituents selected from the group consisting of: alkyl, C₁-C₁₂aryl, substituted cycloalkyl, substituted C₁-C₁₂aryl, aryloxy, alkoxy, trifluoromethyl, cycloalkyl, nitro, cyano, hydroxy, and halogen; and
pharmaceutically acceptable salts, hydrates, solvates and esters thereof.

Particularly preferred among the present Formula I compounds are those in which R¹ is carboxylic acid or sulfonic acid; R, R² and R³ are each independently selected from hydrogen, C₁₋₆alkoxy, tetrazole, -CONR⁵R⁶ where R⁵ and R⁶ are as described in Formula I above, phosphonic acid, phosphinic acid, C₁₋₆alkyl and halogen; m is 0; R⁹ is C₁₋₆alkyl, C₁₋₆alkoxy, halogen, or C₁-C₁₂aryl; and R¹⁰ is phenyl substituted with one or more substituents selected from the group consisting of: alkyl, substituted alkyl, alkoxy, trifluoromethyl, halogen, and hydroxy; and
pharmaceutically acceptable salts, hydrates, solvates and esters thereof.

The most preferred among the present Formula I compounds are those in which R¹ is carboxylic acid or sulfonic acid; R, R² and R³ are each independently selected from hydrogen, C₁₋₆alkoxy, C₁₋₆alkyl and halogen; m is 0; R⁹ is C₁₋₆alkyl or C₁₋₆alkoxy and R¹⁰ is phenyl substituted with from one to three substituents selected from the group consisting of: alkyl, hydroxy, alkoxy, trifluoromethyl and halogen; and
pharmaceutically acceptable salts, hydrates, solvates and esters thereof.

Compounds of Formula I according to the invention are
3-hydroxy-4-{[5-hydroxy-3-methyl-1-(4-methytphenyl)-1H-pyrazol-4-yl]azo}benzoic acid;
4-{[1-(4-benzyloxyphenyl)-5-hydroxy-3-methyl-1H-pyrazol-4-yl]azo}-3-hydroxybenzoic acid;
4-{[1-(4-chlorophenyl)-5-hydroxy-3-methyl-1H-pyrazol-4-yl]azo}-3-hydroxybenzoic acid;
4-{[1-(3-chlorophenyl)-5-hydroxy-3-methyl-1H-pyrazol-4-yl]azo}-3-hydroxybenzoic acid;
4-{[1-(3,4-dimethylphenyl)-5-hydroxy-3-methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-4-hydroxybenzoic acid;
3-hydroxy-4-{[5-hydroxy-3-methyl-1-(3-methylphenyl)-1H-pyrazol-4-yl]azo}benzoic acid;
3-hydroxy-4-{[5-hydroxy-3-methyl-1-(4-trifluoromethylphenyl)-1H-pyrazol-4-yl]azo}benzoic acid;
3-{[1-(3,4-dimethylphenyl)-5-hydroxy-3-methyl-1H-pyrazol-4-yl]azo}-2-hydroxybenzoic acid;
2-{[1-(3,4-dimethylphenyl)-5-hydroxy-3-methyl-1H-pyrazol-4-yl]azo}-3-hydroxybenzoic acid;
5-chloro-3-{[1-(3,4-dimethylphenyl)-5-hydroxy-3-methyl-1H-pyrazol-4-yl]azo}-2-hydroxybenzenesulfonic acid;
3-*tert*-butyl-4-{[1-(3,4-dimethylphenyl)-5-hydroxy-1H-pyrazol-4-yl]azo}-3-hydroxybenzoic acid;
4-{[1-(3,4-dimethylphenyl)-5-hydroxy-3-phenyl-1H-pyrazol-4-yl]azo}-3-hydroxybenzoic acid;
methyl 3-hydroxy-4-{[5-hydroxy-3-methyl-1-(4-methylphenyl)-1H-pyrazol-4-yl]azo}benzoate; and
4-{[1-(4-*tert*-butylphenyl)-5-hydroxy-3-methyl-1H-pyrazol-4-yl]azo}-3-hydroxybenzoic acid;
and pharmaceutically acceptable salts, hydrates, solvates and esters thereof.

This disclosure describes a method of treating thrombocytopenia, which comprises administering to a subject in need thereof an effective amount of a TPO mimetic compound defined by Formula (II) above.

Preferred among the compounds of Formula II are those in which R¹ is carboxylic acid or sulfonic acid; R, R² and R³ are each independently selected from hydrogen, carboxylic acid, C₁-C₁₂aryl, sulfonic acid, tetrazole, -CONR⁵R⁶ where R⁵ and R⁶ are as described in Formula II above, phosphonic acid, phosphinic acid, C₁₋₆alkoxy, nitro, C₁₋₆alkyl and halogen; m is 0; and AR is a cyclic or polycyclic aromatic ring containing from 3 to 14 carbon atoms, optionally containing one or more heteroatoms, provided that when the number of carbon atoms is 3 the aromatic ring contains at least two heteroatoms and when the number of carbon atoms is 4 the aromatic ring at least one heteroatom, and optionally substituted with one or more substituents selected from the group consisting of: alkyl, C₁₋C₁₂ aryl, substituted cycloalkyl, substituted C₁-C₁₂aryl, aryloxy, hydroxy, alkoxy, cycloalkyl, amino, nitro, cyano, and halogen; and pharmaceutically acceptable salts, hydrates, solvates and esters thereof.

Particularly preferred among the compounds of Formula II are those in which R¹ is carboxylic acid or sulfonic acid; R, R² and R³ are each independently selected from hydrogen, C₁₋₆alkoxy, tetrazole, -CONR⁵R⁶ where R⁵ and R⁶ are as described in Formula II above, phosphonic acid, phosphinic acid, C₁₋₆alkyl and halogen; m is 0; and AR is a cyclic or polycyclic aromatic ring containing from 3 to 14 carbon atoms, optionally containing one or more heteroatoms, provided that when the number of carbon atoms is 3 the aromatic ring contains at least two heteroatoms and when the number of carbon atoms is 4 the aromatic ring at least one heteroatom, and optionally substituted with one or more substituents selected from the group consisting of: alkyl, C₁-C₁₂ aryl, substituted C₁₋C₁₂aryl, aryloxy, hydroxy, alkoxy, amino, and halogen; and pharmaceutically acceptable salts, hydrates, solvates and esters thereof.

The most preferred among the compounds of Formula II are those in which R¹ is carboxylic acid or sulfonic acid; R, R² and R³ are each independently selected from hydrogen, C₁₋₆alkoxy, C₁₋₆alkyl and halogen; m is 0; and AR is selected from naphthalene, phenyl and pyrazole, and optionally substituted with from one to three substituents selected from the group consisting of: alkyl, C₁-C₁₂ aryl, substituted C₁₋C₁₂aryl, hydroxy, alkoxy and halogen; and
pharmaceutically acceptable salts, hydrates, solvates and esters thereof.

Preferred among the compounds of Formula II are
3-hydroxy-4-{[5-hydroxy-3-methyl-1-(4-methylphenyl)-1H-pyrazol-4-yl]azo}benzoic acid;
3-hydroxy-4-[(5-hydroxy-3-methyl-1-phenyl-1H-pyrazol-4-yl)azo]benzoic acid;
4-{[1-(4-benzyloxyphenyl)-5-hydroxy-3-methyl-1H-pyrazol-4-yl]azo}-3-hydroxybenzoic acid;
4-{[1-(4-chlorophenyl)-5-hydroxy-3-methyl-1H-pyrazol-4-yl]azo}-3-hydroxybenzoic acid;
4-{[1-(3-chlorophenyl)-5-hydroxy-3-methyl-1H-pyrazol-4-yl]azo}-3-hydroxybenzoic acid;
4-{[1-(3,4-dimethylphenyl)-5-hydroxy-3-methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-4-hydroxybenzoic acid;
3-hydroxy-4-{[5-hydroxy-3-methyl-1-(3-methylphenyl)-1H-pyrazol-4-yl]azo}benzoic acid;
3-hydroxy-4-{[5-hydroxy-3-methyl-1-(4-trifluoromethylphenyl)-1H-pyrazol-4-yl]azo}benzoic acid;
3-{[1-(3,4-dimethylphenyl)-5-hydroxy-3-methyl-1H-pyrazol-4-yl]azo}-2-hydroxybenzoic acid;
2-{[1-(3,4-dimethylphenyl)-5-hydroxy-3-methyl-1H-pyrazol-4-yl]azo}-3-hydroxybenzoic acid;
5-chloro-3-{[1-(3,4-dimethylphenyl)-5-hydroxy-3-methyl-1H-pyrazol-4-yl]azo}-2-hydroxybenzenesulfonic acid;
3-*tert*-butyl-4-{[1-(3,4-dimethylphenyl)-5-hydroxy-1H-pyrazol-4-yl]azo}-3-hydroxybenzoic acid;
4-{[1-(3,4-dimethylphenyl)-5-hydroxy-3-phenyl-1H-pyrazol-4-yl]azo}-3-hydroxybenzoic acid;
methyl 3-hydroxy-4-{[5-hydroxy-3-methyl-1-(4-methylphenyl)-1H-pyrazol-4-yl]azo}benzoate; and
4-{[1-(4-*tert*-butylphenyl)-5-hydroxy-3-methyl-1H-pyrazol-4-yl]azo}-3-hydroxybenzoic acid;
and pharmaceutically acceptable salts, hydrates, solvates and esters thereof.

Compounds of Formula (I) are included in the pharmaceutical compositions of the invention and used in the methods of the disclosure.

Compounds of Formula (II) are included in the pharmaceutical compositions of the invention and used in the methods of the disclosure.

By the term "aryl" as used herein, unless otherwise defined, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbon atoms is 3 the aromatic ring contains at least two heteroatoms and when the number of carbon atoms is 4 the aromatic ring contains at least one heteroatom.

By the term "C₁-C₁₂aryl" as used herein, unless otherwise defined, is meant phenyl, naphthalene, 3,4-methylenedioxyphenyl, pyridine, biphenyl, quinoline, pyrimidine, quinazoline, thiophene, furan, pyrrole, pyrazole, imidazole and tetrazole.

By the term "substituted" as used herein, unless otherwise defined, is meant that the subject chemical moiety has one or more substituents selected from the group consisting of: hydroxyalkyl, alkoxy, acyloxy, alkyl, amino, N-acylamino, hydroxy, -(CH₂)_{g}C(O)OR¹¹, -S(O)ₙR¹², nitro, cyano, halogen, and trifluoromethyl, where g is 0-6, R¹¹ is hydrogen or alkyl, n is 0-2, and R¹² is hydrogen or alkyl.

By the term "alkoxy" as used herein is meant -Oalkyl where alkyl is as described herein including -OCH₃ and -OC(CH₃)₂CH₃.

The term "cycloalkyl" as used herein unless otherwise defined, is meant a nonaromatic, unsaturated or saturated, cyclic or polycyclic C₃-C_{12.}

Examples of cycloalkyl and substituted cycloalkyl substituents as used herein include: cyclohexyl, 4-hydroxy-cyclohexyl, 2-ethylcyclohexyl, propyl 4-methoxycyclohexyl, 4-methoxycyclohexyl, 4-carboxycyctohexyl and cyclopentyl.

By the term "acyloxy" as used herein is meant -OC(O)alkyl where alkyl is as described herein. Examples of acyloxy substituents as used herein include: -OC(O)CH₃, -OC(O)CH(CH₃)₂ and -OC(O)(CH₂)₃CH₃.

By the term "N-acylamino" as used herein is meant -N(H)C(O)alkyl, where alkyl is as described herein. Examples of N-acylamino substituents as used herein include: -N(H)C(O)CH₃, -N(H)C(O)CH(CH₃)₂ and -N(H)C(O)(CH₂)₃CH₃.

By the term "aryloxy" as used herein is meant -OC₆-C₁₂aryl where C₆-C₁₂aryl is phenyl, naphthyl, 3,4-methylenedioxyphenyl, or biphenyl optionally substituted with one or more substituents selected from the group consisting of: alkyl, hydroxyalkyl, alkoxy, trifluoromethyl, acyloxy, amino, N-acylamino, hydroxy, -(CH₂)_{g}C(O)OR¹¹, -S(O)ₙR¹², nitro, cyano, and halogen, where g is 0-6, R¹¹ is hydrogen or alkyl, n is 0-2 and R¹² is hydrogen or alkyl. Examples of aryloxy substituents as used herein include: phenoxy, 4-fluorophenyloxy and biphenyloxy.

By the term "heteroatom" as used herein is meant oxygen, nitrogen or sulfur.

By the term "halogen" as used herein is meant a substituent selected from bromide, iodide, chloride and fluoride.

By the term "alkyl" and derivatives thereof and in all carbon chains as used herein is meant a linear or branched, saturated or unsaturated hydrocarbon chain, and unless otherwise defined, the carbon chain will contain from 1 to 12 carbon atoms. Examples of alkyl substituents as used herein include: -CH₃, -CH₂-CH₃, -CH₂-CH₂-CH₃, -CH(CH₃)₂, -C(CH₃)₃, -(CH₂)₃-CH₃, -CH₂-CH(CH₃)₂, -CH(CH₃)-CH₂-CH₃, -CH=CH₂, and -C≡C-CH₃.

By the term "treating" and derivatives thereof as used herein, is meant prophylatic or therapeutic therapy.

All publications, including but not limited to patents and patent applications, cited in this specification are herein incorporated by reference as though fully set forth.

Compounds of Formula (II) are included in the pharmaceutical compositions of the invention and used in the methods of the disclosure. Where a -COOH or -OH group is present, pharmaceutically acceptable esters can be employed, for example methyl, ethyl, pivaloyloxymethyl, and the like for -COOH, and acetate maleate and the like for -OH, and those esters known in the art for modifying solubility or hydrolysis characteristics for use as sustained release or prodrug formulations.

The novel compounds of Formula II are prepared as shown in Schemes I and II below, or by analogous methods, wherein R, R¹, R², R³, AR and m are as defined in Formula II and provided that the 'R' and m substituents and AR do not include any such substituents that render inoperative the processes of Schemes I and II. All of the starting materials are commercially available or are readily made from commercially available starting materials by those of skill in the art.

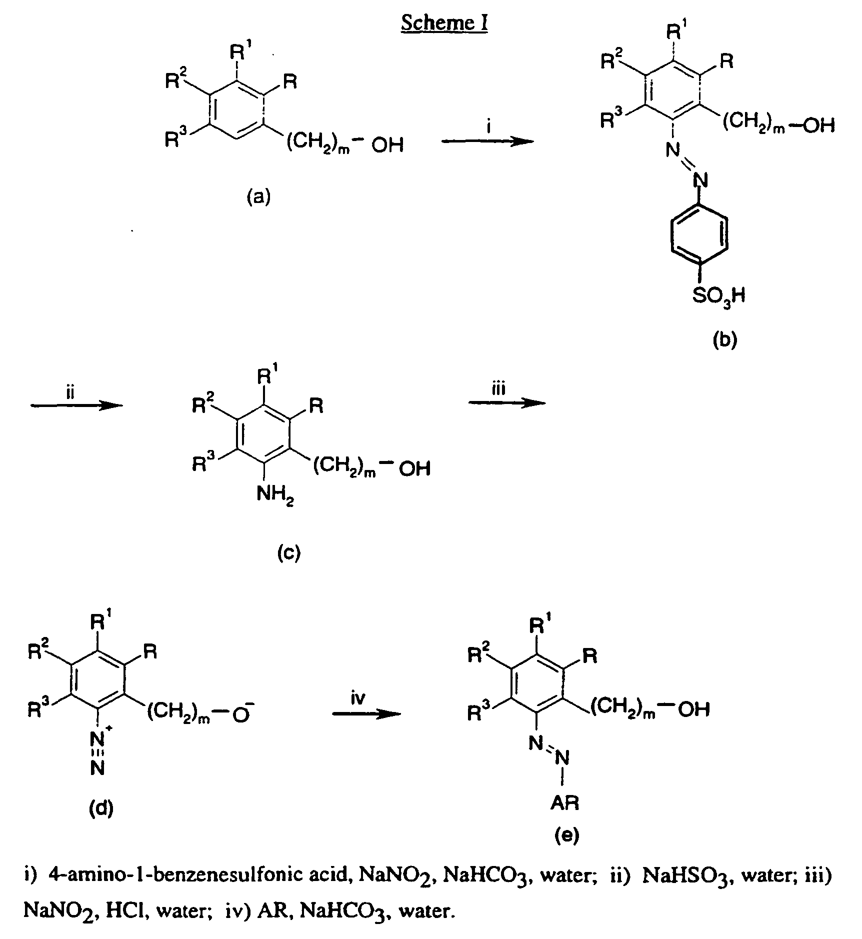

Scheme I outlines the formation of Formula II compounds. As used in scheme I the diazo compound (b) is prepared from the three hydroxybenzene compound (a) by treating (a) with 4-benzenediazonium sulfate in the presence of an appropriate base, preferably sodium hydrogen carbonate. Reduction of compound (b) with sodium hydrogen sulfite in water yielded the 2-aminohydroxybenzene compound (c). Compound (c) is diazotized by reaction with sodium nitrite and an appropriate acid, such as nitric acid, sulfuric acid or, preferably hydrochloric acid, in an appropriate aqueous solvent, such as water or, preferably an ethanol-water mixture to produce diazonium compound (d). Compound (e) is prepared by reacting compound (d) in a coupling reaction with an appropriate aryl species in the presence of a base, preferably sodium hydrogen carbonate, or an acid, preferably hydrochloric acid.

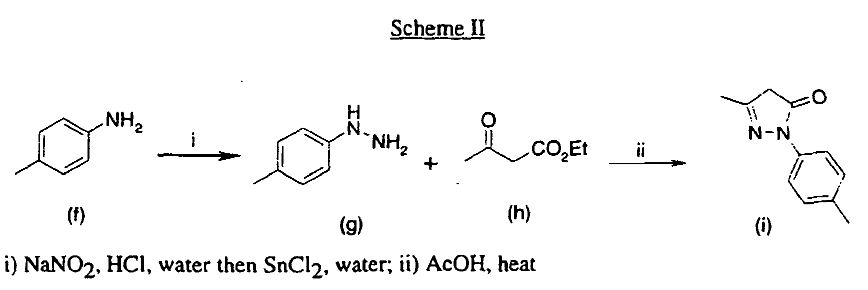

Scheme II outlines the formation of pyrazoles for use in scheme I. An amine such as 4-methylaniline, compound (f), is diazotized by the action of sodium nitrite and an appropriate acid such as hydrochloric acid, nitric acid or sulfuric acid in an appropriate aqueous solvent system such as water or ethanol-water mixtures then reduced *in situ* by tin chloride to afford hydrazine, compound (g). The hydrazine is then condensed with a beta-keto ester such as ethyl acetoacetate, compound (h), in an appropriate solvent such as acetic acid or ethanol at an appropriate temperature typically 0-100° to give the corresponding pyrazole, compound (I) as described herein.

The treatment of thrombocytopenia, as described herein, is accomplished by enhancing the production of platelets.

By the term "co-administering" and derivatives thereof as used herein is meant either simultaneous administration or any manner of separate sequential administration of a TPO mimetic compound, as described herein, and a further active ingredient or ingredients, known to treat thrombocytopenia, including chemotherapy-induced thrombocytopenia and bone marrow transplantation and other conditions with depressed platelet production. Preferably, if the administration is not simultaneous, the compounds are administered in a close time proximity to each other. Furthermore, it does not matter if the compounds are administered in the same dosage form, e.g. one compound may be administered topically and another compound may be administered orally.

Because the pharmaceutically active compounds of the present invention are active as TPO mimetics they exhibit therapeutic utility in treating thrombocytopenia and other conditions with depressed platelet production.

In determining potency as TPO mimetics, the following assays were employed:

### Luciferase Assay

Compounds of the present invention were tested for potency as mimetics of the TPO receptor in a Luciferase assay such as described in Lamb, et al., Nucleic Acids Research 23: 3283-3289 (1995) and Seidel, et al., Proc. Natl. Acad. Sci., USA 92: 3041-3045 (1995) by substituting a TPO-responsive BaF3 cell line (Vigon et al. Proc. Natl. Acad. Sci. USA 1992, 89, 5640-5644) for the HepG2 cells utilized therein. The murine BaF3 cells express TPO receptors and closely match the pattern of STAT (signal transducers and activators of transcription) activation observed in primary murine and human bone marrow cells.

Some of the most preferred compounds of this invention were also active in an in vitro proliferation assay using the murine 32D-mpl cell line (Bartley, T. D. et al., Cell, 1994, 77, 1117-1124). 32D-mpl cells express Tpo-R and their survival is dependent on the presence of TPO. Likewise, some of the most preferred compounds of this invention were also positive in stimulating the maturation of megakaryocytes from human bone marrow cells. In this assay, purified human CD34+ progenitor cells were incubated in liquid culture with test compounds for 10 days and the number of cells expressing the transmembrane glycoprotein CD41 (gpIIb), a megakaryocytic marker, was then measured by flow cytometry (see Cwirla, S. E. et al Science, 1997, 276, 1696-1699).

The pharmaceutically active compounds within the scope of this invention are useful as TPO mimetics in mammals, including humans, in need thereof.

Some of the preferred compounds within the scope of the invention showed activation from about 4% to 100% control at a concentration of 0.1-10 uM in the luciferase assay. The preferred compounds of the invention also promoted the proliferation of 32D-mpl cells at a concentration of 0.1 to 100 uM. The preferred compounds of the invention also showed activity in the CD41 megakaryocytic assay at a concentration of 0.1 to 30 uM.

The present invention can be used in a method of treating thrombocytopenia and other conditions with depressed platelet production, which comprises administering a compound of Formula (II), as defined above, and pharmaceutically acceptable salts, hydrates, solvates and esters thereof, in a quantity effective to enhance platelet production. The compounds of Formula (II) also can be used in a method of treating the above indicated disease states because of their demonstrated ability to act as TPO mimetics. The drug may be administered to a patient in need thereof by any conventional route of administration, including, but not limited to, intravenous, intramuscular, oral, subcutaneous, intradermal, and parenteral.

The pharmaceutically active compounds of the present invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral or parenteral products.

Doses of the presently invented pharmaceutically active compounds in a pharmaceutical dosage unit as described above will be an efficacious, nontoxic quantity preferably selected from the range of 0.001 - 100 mg/kg of active compound, preferably 0.001 - 50 mg/kg. When treating a human patient in need of a TPO mimetic, the selected dose is administered preferably from 1-6 times daily, orally or parenterally. Preferred forms of parenteral administration include topically, rectally, transdermally, by injection and continuously by infusion. Oral dosage units for human administration preferably contain from 0.05 to 3500 mg of active compound. Oral administration, which uses lower dosages is preferred. Parenteral administration, at high dosages, however, also can be used when safe and convenient for the patient.

Optimal dosages to be administered may be readily determined by those skilled in the art, and will vary with the particular TPO mimetic in use, the strength of the preparation, the mode of administration, and the advancement of the disease condition. Additional factors depending on the particular patient being treated will result in a need to adjust dosages, including patient age, weight, diet, and time of administration.

The method usable in this disclosure inducing TPO mimetic activity in mammals, including humans, comprises administering to a subject in need of such activity an effective TPO mimetic amount of a pharmaceutically active compound of the present invention.

The invention also provides for the use of a compound of Formula (II) in the manufacture of a medicament for use as a TPO mimetic.

The invention also provides for the use of a compound of Formula (II) in the manufacture of a medicament for use in therapy, e.g. as a TPO mimetic.

The invention also provides for the use of a compound of Formula (II) in the manufacture of a medicament for use in administering a therapeutically effective amount of the compound to a mammal and enhancing platelet production in the mammal, wherein the mammal (e.g. human) is in need of enhanced platelet production.

The invention also provides for the use of a compound of Formula (II) in the manufacture of a medicament for use in treating thrombocytopenia.

The invention also provides for a pharmaceutical composition for use as a TPO mimetic which comprises a compound of Formula (II) and a pharmaceutically acceptable carrier.

The invention also provides for a pharmaceutical composition for use in the treatment of thrombocytopenia which comprises a compound of Formula (II) and a pharmaceutically acceptable carrier.

The invention also provides for a pharmaceutical composition for use in enhancing platelet production which comprises a compound of Formula (II) and a pharmaceutically acceptable carrier.

No unacceptable toxicological effects are expected when compounds of the invention are administered in accordance with the present invention.

In addition, the pharmaceutically active compounds of the present invention can be co-administered with further active ingredients, such as other compounds known to treat thrombocytopenia, including chemotherapy-induced thrombocytopenia and bone marrow transplantation and other conditions with depressed platelet production, or compounds known to have utility when used in combination with a TPO mimetic.

Contemplated Equivalents - It will be appreciated by the person of ordinary skill in the art that the compounds of Formula I and II may also exist in tautomeric forms, wherein the double bond that is drawn between the two nitrogen atoms exists between the lower nitrogen atom and the AR substituent. Tautomeric forms of the compounds of Formula I and II are exemplified by the following Formula III 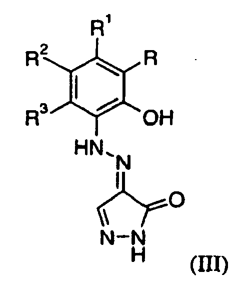
where the 'R' groups are as defined above. All such compounds are included in the scope of the invention and inherently included in the definition of the compounds of Formulas I and II.

Without further elaboration, it is believed that one skilled in the art can, using the preceding description, utilize the present invention to its fullest extent. The following Examples are, therefore, to be construed as merely illustrative and not a limitation of the scope of the present invention in any way.

### Experimental Details

### Example 1

### Preparation of 3-hydroxy-4-{[5-hydroxy-3-methyl-1-(4-methylphenyl)-1H-pyrazol-4-yl]azo}benzoic acid;

A solution of 4-amino-3-hydroxybenzoic acid (0.77g, 0.005 mol.) in 1N aqu. hydrochloric acid (15.0 mL) was cooled to 0°C and treated slowly with a solution of sodium nitrite (0.38 g; 0.0055 mol.) in water (5.0 mL). After addition the solution was stirred at 0°C for 10 min. then 3-methyl-1-(4-methylphenyl)-3-pyrazolin-5-one (0.94 g; 0.005 mol.) was added in one portion. Ethanol (15.0 mL) was added followed by sat. aqu. sodium hydrogen carbonate until the pH of the solution was 8 (ca. 10 mL needed). The red solution was then stirred at room temperature for 16h.

The mixture was filtered and the solid dissolved in 10% aqu. sodium hydroxide (50.0 mL). The red solution was extracted twice with ethyl acetate then acidified with 6N aqu. hydrochloric acid and filtered to give the title compound as a red solid (1.67 g; 95%). MS(ES) m/z 351 [M-H].

### Example 2

### Preparation of 3-hydroxy-4-[(5-hydroxy-3-methyl-1-phenyl-1H-pyrazol-4-yl)azo]benzoic acid:

Following the procedure of Example 1, except substituting 3-methyl-1-phenyl-3-pyrazolin-5-one for 3-methyl-1-(4-methylphenyl)-3-pyrazolin-5-one, the title compound was prepared as a red solid. MS(ES) m/z 351 [M-H].

### Example 3

### Preparation of 4-{[1-(4-benzyloxyphenyl)-5-hydroxy-3-methyl-1H-pyrazol-4-yl]azo}-3-hydroxybenzoic acid;

### a) 4-Benzyloxyphenylhydrazine

A solution of 4-benzyloxyaniline hydrochloride (11.3 g; 0.048 mole) in concentrated hydrochloric acid (40.0 mL) was cooled to 0° then treated dropwise with a solution of sodium nitrite (3.28 g; 0.048 mole) in water (20.0 mL). The mixture was stirred at 0° for a further 10 min. then poured into a cold (-10°) solution of tin dichloride hydrate (40.0g; 0.18 mole) in concentrated hydrochloric acid (40.0 mL). The mixture was allowed to warm to room temperature with stirring for 1h.

The mixture was basified with 10% aqu. sodium hydroxide, ethyl acetate (1L) was added and the mixture filtered to remove unwanted tin residues. The organic layer was then dried and evaporated to afford the title compound as a yellow solid (6.9 g; 67%). mp 105-1070.

### b) 1-(4-Benzyloxyphenyl)-3-methyl-3-pyrazolin-5-one

A solution of the compound from Example 3a) (2.6 g; 0.012 mol.) and ethyl acetoacetate (1.60 mL; 0.012 mol.) in glacial acetic acid (50.0 mL) was stirred and heated at 100° for 24h.

The solvent was evaporated and the product purified by chromatography (silica gel, 50% ethyl acetate/hexanes), the title compound was prepared (2.0 g; 60%). MS(ES) m/z 281 [M+H].

### c) 4-{[1-(4-Benzyloxyphenyl)-5-hydroxy-3-methyl-1H-pyrazol-4-yl]azo}-3-hydroxybenzoic acid

Following the procedure of Example 1, except substituting 1-(4-benzyloxyphenyl)-3-methyl-3-pyrazolin-5-one for 3-methyl-1-(4-methylphenyl)-3-pyrazolin-5-one, and followed by chromatography (silica gel, 10% methanol/ethyl acetate), the title compound was prepared as a brown powder (19%). mp = 258-260°C (decomp).Anal. (C₂₄H₂₀N₄O₅.CH₃OH) calcd: C, 63.00; H, 5.08; N, 11.76 found: C, 63.17; H, 4.64; N, 11.47.

### Example 4

### Preparation of 4-{[1-(4-chlorophenyl)-5-hydroxy-3-methyl-1H-pyrazol-4-yl]azo}-3-hydroxybenzoic acid;

Following the procedure of Example 1, except substituting 1-(4-chlorophenyl)-3-methyl-3-pyrazolin-5-one for 3-methyl-1-(4-methylphenyl)-3-pyrazolin-5-one, the title compound was prepared as a red solid. MS(ES) m/z 371, 373 [M-H].

### Example 5

### Preparation of 4-{[1-(3-chlorophenyl)-5-hydroxy-3-methyl-1H-pyrazol-4-yl]azo}-3-hydroxybenzoic acid:

Following the procedure of Example 1, except substituting 1-(3-chlorophenyl)-3-methyl-3-pyrazolin-5-one for 3-methyl-1-(4-methylphenyl)-3-pyrazolin-5-one, the title compound was prepared as a red solid. MS(ES) m/z 371, 373 [M-H].

### Example 6

### Preparation of 4-{[1-(3,4-dimethylphenyl)-5-hydroxy-3-methyl-1H-pyrazol-4-yl]azo}-3-hydroxybenzoic acid;

### a) 1-(3,4-Dimethylphenyl)-3-phenyl-3-pyrazolin-5-one

Following the procedure of Example 3b), except substituting 3,4-dimethylphenylhydrazine for 4-benzyloxyphenylhydrazine, the title compound was prepared (16.0 g; 61 %). MS(ES) m/z 265 [M+H].

### b) 4-{[1-(3,4-Dimethylphenyl)-5-hydroxy-3-methyl-1H-pyrazol-4-yl]azo}-3-hydroxybenzoic acid

Following the procedure of Example 1, except substituting 1-(3,4-dimethylphenyl)-3-methyl-3-pyrazolin-5-one for 3-methyl-1-(4-methylphenyl)-3-pyrazolin-5-one, the title compound was prepared as an orange solid (1.5 g, 82%). ¹H NMR (400 MHz, d6-DMSO) δ 13.5 br s, 1H), 11.0 (s, 1H), 7.70 (m, 2H), 7.61 (dd, J = 8.2 and 2.1 Hz, 1H), 7.53 (m, 2H), 8.20 (d, J = 8.2 Hz, 1H), 2.30 (s, 3H), 2.27 (s, 3H), 2.22 (s, 3H).

### Example 7

### Preparation of 3-{[1-(3,4-dimethylphenyl)-5-hydroxy-3-methyl-1H-pyrazol-4-yl]azo}-4-hydroxybenzoic acid;

Following the procedure of Example 1, except substituting 1-(3,4-dimethylphenyl)-3-methyl-3-pyrazolin-5-one for 3-methyl-1-(4-methylphenyl)-3-pyrazolin-5-one and 3-amino-4-hydroxybenzoic acid for 4-amino-3-hydroxybenzoic acid, the title compound was prepared as an orange solid (1.5 g, 82%). ¹H NMR (400 MHz, d6-DMSO) δ 13.5 br s, 1H), 11.7 (s, 1H), 8.13 (d, J = 2.0 Hz, 1H), 7.69-7.59 (m, 3H), 7.17 (d, J = 8.4 Hz, 1H), 7.10 (d, J = 8.5 Hz, 1H), 2.30 (s, 3H), 2.25 (s, 3H), 2.20 (s, 3H).

### Example 8

### Preparation of 3-hydroxy-4-{[5-hydroxy-3-methyl-1-(3-methylphenyl)-1H-pyrazol-4-yl]azo}benzoic acid:

### a) 1-(3-Methylphenyl)-3-phenyl-3-pyrazolin-5-one

Following the procedure of Example 3b), except substituting 3-dimethylphenylhydrazine for 4-benzyloxyphenylhydrazine, the title compound was prepared (2.8 g; 90%). MS(ES) m/z 189 [M+H].

### b) 3-Hydroxy-4-{[5-hydroxy-3-methyl-1-(3-methylphenyl)-1H-pyrazol-4-yl]azo}benzoic acid

Following the procedure of Example 1, except substituting 1-(3-methylphenyl)-3-methyl-3-pyrazolin-5-one for 3-methyl-1-(4-methylphenyl)-3-pyrazolin-5-one, the title compound was prepared as an orange solid (0.87 g; 50%). MS(ES) m/z 353 [M+H].

### Example 9

### Preparation of 3-hydroxy-4-{[5-hydroxy-3-methyl-1-(4-trifluoromethylphenyl)-1H-pyrazol-4-yl]azo}benzoic acid;

### a) 3-Phenyl-1-(4-trifluoromethylphenyl)-3-pyrazolin-5-one

Following the procedure of Example 3b), except substituting 4-trifluoromethylphenylhydrazine for 4-benzyloxyphenylhydrazine, the title compound was prepared (3.3 g; 92%). MS(ES) m/z 243 [M+H].

### b) 3-Hydroxy-4-{[5-hydroxy-3-methyl-1-(4-trifluoromethylphenyl)-1H-pyrazol-4-yl]azo}benzoic acid

Following the procedure of Example 1, except substituting 3-phenyl-1-(4-trifluoromethylphenyl)-3-pyrazolin-5-one for 3-methyl-1-(4-methylphenyl)-3-pyrazolin-5-one, the title compound was prepared as an orange solid (0.86 g g; 35%). MS(ES) m/z 407 [M+H].

### Example 10

### Preparation of 3-{[1-(3,4-dimethylphenyl)-5-hydroxy-3-methyl-1H-pyrazol-4-yl]azo}-2-hydroxybenzoic acid;

Following the procedure of Example 1, except substituting 1-(3,4-dimethylphenyl)-3-methyl-3-pyrazolin-5-one for 3-methyl-1-(4-methylphenyl)-3-pyrazolin-5-one and 3-amino-2-hydroxybenzoic acid for 4-amino-3-hydroxybenzoic acid, the title compound was prepared as an orange solid (0.40 g, 32%). ¹H NMR (400 MHz, d6-DMSO) δ 7.83 (m, 2H), 7.59 (d, J = 7.8 Hz, 1H), 7.50 (d, J = 7.8 Hz, 1H), 7.09 (d, J = 8.0 Hz, 1H), 6.60 (t, J = 7.8 Hz, 1H), 2.29 (s, 3H), 2,24 (s, 3H), 2.21 (s, 3H).

### Example 11

### Preparation of 5-chloro-3-{[1-(3,4-dimethylphenyl)-5-hydroxy-3-methyl-1H-pyrazol-4-yl]azo}-2-hydroxybenzenesulfonic acid;

Following the procedure of Example 1, except substituting 1-(3,4-dimethylphenyl)-3-methyl-3-pyrazol in-5-one for 3-methyl-1-(4-methylphenyl)-3-pyrazolin-5-one and 3-amino-5-chloro-2-hydroxybenzenesulfonic acid for 4-amino-3-hydroxybenzoic acid, the title compound was prepared as a red solid (0.74 g, 34%). mp 240°C (decomp). MS(ES) m/z 437, 435 [M-H].

### Example 12

### Preparation of 4-{[3-tert-butyl-1-(3,4-dimethylphenyl)-5-hydroxy-1H-pyrazol-4-yl]azo}-3 hydroxybenzoic acid:

### a) 3-tert-Butyl-1-(3,4-dimethylphenyl)-3-pyrazolin-5-one

Following the procedure of Example 3b), except substituting 3,4-dimethylphenylhydrazine for 4-benzyloxyphenylhydrazine and ethyl *tert*-butylacetate for ethyl acetoacetate, the title compound was prepared (25.1 g; 99%). MS(ES) m/z 245 [M+H].

### b) 4-{[3-tert-Butyl-1-(3,4-dimethylphenyl)-5-hydroxy-1H-pyrazol-4-yl]azo}-3-hydroxybenzoic acid

Following the procedure of Example 1, except substituting 3-*tert*-butyl-1-(3,4-dimethylphenyl)-3-pyrazolin-5-one for 3-methyl-1-(4-methylphenyl)-3-pyrazolin-5-one, the title compound was prepared (0.71 g; 70%) as an orange solid, MS(ES) m/z 409 [M+H].

### Example 13

### Preparation of 4-{[1-(3,4-dimethylphenyl)-5-hydroxy-3-phenyl-1H-pyrazol-4-yl]azo}-3-hydroxybenzoic acid;

### a) 1-(3,4-Dimethylphenyl)-3-phenyl-3-pyrazolin-5-one

Following the procedure of Example 3b), except substituting 3,4-dimethylphenylhydrazine for 4-benzyloxyphenylhydrazine and ethyl benzoylacetate for ethyl acetoacetate, the title compound was prepared (16.0 g; 61 %). MS(ES) m/z 265 [M+H].

### b) 4-{[1-(3,4-Dimethylphenyl)-5-hydroxy-3-phenyl-1H-pyrazol-4-yl]azo}-3-hydroxybenzoic acid

Following the procedure of Example 1, except substituting 1-(3,4-dimethylphenyl)-3-phenyl-3-pyrazolin-5-one for 3-methyl-1-(4-methylphenyl)-3-pyrazolin-5-one, the title compound was prepared (1.0 g; 78%) as a red solid, MS(ES) m/z 429 [M+H].

### Example 14

### Preparation of methyl 4-{[5-hydroxy-3-methyl-1-(4-methylphenyl)-1H-pyrazol-4-yl]azo}-3-hydroxybenzoate

Following the procedure of Example 1, except substituting 1-(3,4-dimethylphenyl)-3-methyl-3-pyrazolin-5-one for 3-methyl-1-(4-methylphenyl)-3-pyrazolin-5-one and methyl 4-amino-3-hydroxybenzoate for 4-amino-3-hydroxybenzoic acid, the title compound was prepared as a red solid (0.059 g, 10%). MS(ES) m/z 367 [M+H].

### Example 15

### Preparation of 4-{[1-(4-tert-butylphenyl)-5-hydroxy-3-methyl-1H-pyrazol-4-yl]azo}-3-hydroxybenzoate

Following the procedure of Example 1, except substituting 1-(4-tert-butylphenyl)-3-methyl-3-pyrazolin-5-one for 3-methyl-1-(4-methylphenyl)-3-pyrazolin-5-one, the title compound was prepared as an orange solid (0.895 g, 45%). MS(ES) m/z 395 [M+H], Anal. (C₂₁H₂₂N₄O₄) calcd: C, 63.95; H, 5.62; N, 14.20 found: C, 63.65; H, 5.75; N, 13.83.

### Example 16 - Capsule Composition

An oral dosage form for administering a presently invented agonist of the TPO receptor is produced by filing a standard two piece hard gelatin capsule with the ingredients in the proportions shown in Table I, below.

**Table I**

| INGREDIENTS | AMOUNTS |
|---|---|
| 3-hydroxy-4-{[5-hydroxy-3-methyl-1-(4-methylphenyl)-1H-pyrazol-4-yl]azo}benzoic acid (Compound 1) | 25 mg |
| Lactose | 55 mg |
| Talc | 16 mg |
| Magnesium Stearate | 4 mg |

### Example 17 - Injectable Parenteral Composition

An injectable form for administering a presently invented agonist of the TPO receptor is produced by stirring 1.5% by weight of 4-{[1-(4-benzyloxyphenyl)-5-hydroxy-3-methyl-1H-pyrazol-4-yl]azo}-3-hydroxybenzoic acid, monosodium salt (Compound 2) in 10% by volume propylene glycol in water.

### Example 18 - Tablet Composition

The sucrose, calcium sulfate dihydrate and a presently invented agonist of the TPO receptor, as shown in Table II below, are mixed and granulated in the proportions shown with a 10% gelatin solution. The wet granules are screened, dried, mixed with the starch, talc and stearic acid, screened and compressed into a tablet.

**Table II**

| INGREDIENTS | AMOUNTS |
|---|---|
| 4-{[1-(3,4-dimethylphenyl)-5-hydroxy-3-methyl-1H-pyrazol-4-yl]azo}-3-hydroxybenzoic acid (Compound 6) | 20 mg |
| calcium sulfate dihydrate | 30 mg |
| sucrose | 4 mg |
| starch | 2 mg |
| talc | 1 mg |
| stearic acid | 0.5 mg |

Preferred among the compounds of the present invention are the following;
3-hydroxy-4-{[5-hydroxy-3-methyl-1-(4-methylphenyl)-1H-pyrazol-4-yl]azo}benzoic acid; 4-{[1-(4-benzyloxyphenyl)-5-hydroxy-3-methyl-1H-pyrazol-4-yl]azo}-3-hydroxybenzoic acid;
4-{[1-(4-tert-butylphenyl)-5-hydroxy-3-methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-2-hydroxybenzoic acid; and
4-{[1-(3,4-dimethylphenyl)-5-hydroxy-3-methyl-1H-pyrazol-4-yl]azo}-3-hydroxybenzoic acid.

The compound 4-{[1-(3,4-dimethylphenyl)-5-hydroxy-3-methyl-1H-pyrazol-4-y)]azo}-3-hydroxybenzoic acid demonstrated an activity of, EC50= 1.8 uM, 50%TPO in the above luciferase assay.

While the preferred embodiments of the invention are illustrated by the above, it is to be understood that the invention is not limited to the precise instructions herein disclosed and that the right to all modifications coming within the scope of the following claims is reserved.

## Claims

1. The use of a compound of Formula (II) 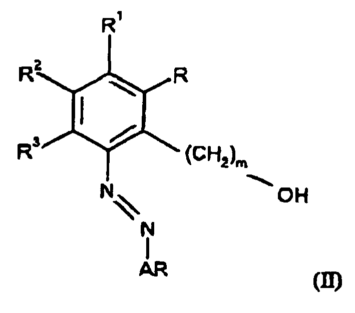 wherein:
R, R¹, R² and R³ are each independently selected from hydrogen, C₁₋₆alkyl, C₁₋₆alkoxy, -(CH₂)ₚOR⁴, -C(O)OR⁴, nitro, cyano, halogen, aryl, -S(O)ₙR⁴. cycloalkyl, -CONR⁵R⁶, -NR⁵R⁶, phosphonic acid, sulfonic acid, phosphinic acid and -SO₂NR⁵R⁶,
where
p is 0-6;
n is 0-2;
R⁴ is hydrogen, alkyl, cycloalkyl, C₁-C₁₂aryl, substituted alkyl, substituted cycloalkyl and substituted C₁-C₁₂aryl and
R⁵ and R⁶ are each independently selected from hydrogen, alkyl, C₃₋₆cycloalkyl, aryl or R⁵ and R⁶ taken together with the nitrogen to which they are attached represent a 5 to 6 member saturated ring containing up to one other heteroatom selected from oxygen and nitrogen;
m is 0-6; and
AR is cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, aryl, substituted cycloalkyl, substituted aryl, aryloxy, hydroxy, alkoxy, cycloalkyl, acyloxy, ammo, N-acylamino, nitro, cyano, halogen, -C(O)OR⁴, -C(O)NR⁷R⁸, -S(O)₂NR⁷R⁸, -S(O)ₙR⁴, and alkyl substituted with one or more substituents selected from the group consisting of: alkoxy, acyloxy, aryl, substituted aryl, amino, N-acylamino, oxo, hydroxy, cycloalkyl, substituted cycloalkyl, -C(O)OR⁴, -C(O)NR⁷R⁸, -S(O)₂NR⁷R⁸, -S(O)ₙR⁴, aryloxy, nitro, cyano, and halogen,
where
R⁴ is hydrogen, alkyl, cycloalkyl, C₁-C₁₂aryl, substituted alkyl, substituted cycloalkyl and substituted C₁-C₁₂aryl; and
R⁷ and R⁸ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴. -S(O)ₙR⁴, -C(O)NR⁴R⁴, -S(O)₂NR⁴R⁴, nitro, cyano, cycloalkyl, substituted cycloalkyl, halogen, C₁-C₁₂aryl, and substituted C₁-C₁₂aryl,
or R⁷ and R⁸ taken together with the nitrogen to which they are attached represent a 5 to 6 member saturated ring containing up to one other heteroatom selected from oxygen and nitrogen,
where R⁴ is as described above and n is 0-2; and
pharmaceutically acceptable salts, hydrates, solvates and esters thereof;
and wherein:
by the term "aryl", unless otherwise defined, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbon atoms is 3 the aromatic ring contains at least two heteroatoms and when the number of carbon atoms is 4 the aromatic ring contains at least one heteroatom;
by the term "C₁-C₁₂aryl", unless otherwise defined, is meant phenyl, naphthalene, 3,4-methylenedioxyphenyl, pyridine, biphenyl, quinoline, pyrimidine, quinazoline, thiophene, furan, pyrrole, pyrazole, imidazole and tetrazole;
by the term "substituted", unless otherwise defined, is meant that the subject chemical moiety has one or more substituents selected from the group consisting of: hydroxyalkyl, alkoxy, acyloxy, alkyl, amino, N-acylamino, hydroxy, -(CH₂)_{g}C(O)OR¹¹, -S(O)ₙR¹², nitro, cyano, halogen, and trifluoromethyl, where g is 0-6, R¹¹ is hydrogen or alkyl, n is 0-2, and R¹² is hydrogen or alkyl;
by the term "alkoxy" is meant -Oalkyl where alkyl is as described herein;
by the term "cycloalkyl", unless otherwise defined, is meant a nonaromatic, unsaturated or saturated, cyclic or polycyclic C₃-C₁₂;
by the term "acyloxy" is meant -OC(O)alkyl where alkyl is as described herein;
by the term "N-acylamino" is meant -N(H)C(O)alkyl, where alkyl is as described herein;
by the term "aryloxy" is meant -OC₆-C₁₂aryl where C₆-C₁₂aryl is phenyl, naphthyl, 3,4-methylenedioxyphenyl, or biphenyl optionally substituted with one or more substituents selected from the group consisting of: alkyl, hydroxyalkyl, alkoxy, trifluoromethyl, acyloxy, amino, N-acylamino, hydroxy, -(CH₂)_{g}C(O)OR¹¹, -S(O)ₙR)², nitro, cyano, and halogen, where g is 0-6, R¹¹ is hydrogen or alkyl, n is 0-2 and R¹² is hydrogen or alkyl;
by the term "heteroatom" is meant oxygen, nitrogen or sulfur,
by the term "halogen" as used herein is meant a substituent selected from bromide, iodide, chloride and fluoride; and
by the term "alkyl" and derivatives thereof and in all carbon chains as used herein is meant a linear or branched, saturated or unsaturated hydrocarbon chain, and unless otherwise defined the carbon chain will contain from 1 to 12 carbon atoms;
in the manufacture of a medicament for use in treating thrombocytopenia.

2. The use as claimed in claim 1 wherein, in the compound of Formula (II),
R¹ is carboxylic acid or sulfonic acid;
R, R² and R³ are each independently selected from hydrogen, C₁₋₆alkoxy, tetrazole, -CONR⁵R⁶, phosphonic acid, phosphinic acid, C₁₋₆alkyl and halogen;
m is 0; and
AR is cyclic or polycyclic aromatic ring containing from 3 to 14 carbon atoms, optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, C₁-C₁₂aryl, substituted C₁-C₁₂aryl, aryloxy, hydroxy, alkoxy, amino, and halogen.

3. The use as claimed in claim 1 wherein, in the compound of Formula (II),
R¹ is carboxylic acid or sulfonic acid;
R, R² and R³ are each independently selected from hydrogen, C₁₋₆alkoxy, C₁₋₆alkyl and halogen;
m is 0; and
AR is selected from naphthalene, phenyl and pyrazole, and optionally substituted with from one to three substituents selected from the group consisting of; alkyl, C₁-C₁₂aryl, substituted C₁-C₁₂aryl, hydroxy, alkoxy and halogen.

4. The use as claimed in claim 1 wherein, in the compound of Formula (II),
R¹ is carboxylic acid or sulfonic acid;
R, R² and R³ are each independently selected from hydrogen, C₁₋₆alkoxy, C₁₋₆alkyl and halogen;
m is 0; and
AR is pyrazole optionally substituted with from one to three substituents selected from the group consisting of: alkyl, C₁-C₁₂aryl, substituted C₁-C₁₂ aryl, hydroxy, alkoxy and halogen.

5. The use as claimed in Claim 1 wherein the compound is selected from
3-hydroxy-4-{[5-hydroxy-3-methyl-1-(4-methylphenyl)-1H-pyrazol-4-yl]azo}benzoic acid;
3-hydroxy-4-[(5-hydroxy-3-methyl-1-phenyl-1H-pyrazol-4-yl)azo]benzoic acid;
4-{[1-(4-benzyloxyphenyl)-5-hydroxy-3-methyl-1H-pyrazol-4-yl]azo}-3-hydroxybenzoic acid;
4-{[1-(4-chlorophenyl)-5-hydroxy-3-methyl-1H-pyrazol-4-yl]axo}-3-hydroxybenzoic acid;
4-{[1-(3-chlorophenyl)-5-hydroxy-3-methyl-1H-pyrazol-4-yl]azo)-3-hydroxybenzoic acid;
4-{[1-(3,4-dimethylphenyl)-5-hydroxy-3-methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-4-hydroxybenzoic acid;
3-hydroxy-4-{[5-hydroxy-3-methyl-1-(3-methylphenyl)-1H-pyrazol-4-yl]azo}benzoic acid;
3-hydroxy-4-{[5-hydroxy-3-methyl-1-(4-trifluoromethylphenyl-1H-pyrazol-4-yl]azo}benzoic acid;
3-{[1-(3,4-dimethylphenyl)-5-hydroxy-3-methyl-1H-pyazol]-4-yl]azo}-2-hydroxybenzoic acid;
2-{[1-(3,4-dimethylphenyl)-5-hydroxy-3-methyl-1H-pyrazol-4-yl]azo}-3-hydroxybenzoic acid;
5-chloro-3-{[1-(3,4-dimemylphenyl)-5-hydroxy-3-methyl-1H-pyrazol-4-yl]azo}-2-hydroxybenzenesulfonic acid;
3-*tert*-butyl-4-{[1-(3,4-dimethylphenyl)-5-hydroxy-1H-pyrazol-4-yl]azo}-3-hydroxybenzoic acid;
4-{[1-(3,4-dimethylphenyl)-5-hydroxy-3-phenyl-1H-pyrazol-4-yl]azo}-3-hydroxybenzoic acid;
methyl 3-hydroxy-4-{[5-hydroxy-3-methyl-1-(4-methylphenyl)-1H-pyrazol-4-yl]azo}benzoate; and
4-{[1-(4-*tert*-butylphenyl)-5-hydroxy-3-methyl-1H-pyrazol-4-yl]azo}-3-hydroxybenzoic acid;
and pharmaceutically acceptable salts, hydrates, solvates and esters thereof.

6. The use of a compound of Formula (II), as defined in any preceding claim, in the manufacture of a medicament,
wherein the medicament is for use in administering a therapeutically effective amount of the compound to a mammal and enhancing platelet production in the mammal,
and wherein the mammal, including a human, is in need of enhanced platelet production.

7. The use of a compound of Formula (II), as described in any preceding claim, in the manufacture of a medicament for use in administering an effective amount of the compound to and agonizing the TPO receptor in a subject.

8. A pharmaceutical composition for use in the treatment of thrombocytopenia which comprises a compound of Formula (II), as defined in any preceding claim, and a pharmaceutically acceptable carrier.

9. A pharmaceutical composition for use in administering to and enhancing platelet production in a mammal, including a human, in need of enhanced platelet production,
which composition comprises a therapeutically effective amount of a compound of Formula (II), as described in any preceding claim, and a pharmaceutically acceptable carrier.

10. The use or composition as claimed in any preceding claim wherein the medicament or composition is for oral administration.

11. The use or composition as claimed in any preceding claim wherein the medicament or composition is for parenteral administration.

12. A medicament for use in therapy, manufactured using and comprising a compound represented by the following Formula (I) 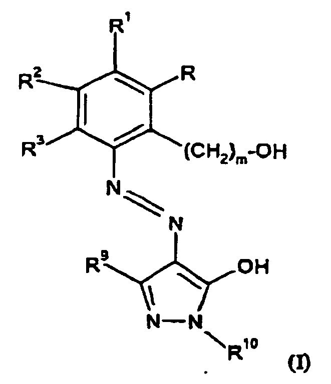 wherein:
R, R¹, R², R³ and R⁹ are each independently selected from hydrogen, C₁₋₆alkyl, C₁₋₆alkoxy, -(CH₂)ₚOR⁴, -C(O)OR⁴, nitro, cyano, halogen, aryl, -S(O)ₙR⁴, cycloalkyl, -CONR⁵R⁶, phosphonic acid, sulfonic acid, phosphinic acid and -SO₂NR⁵R⁶,
where
p is 0-6;
n is 0-2;
R⁴ is hydrogen, alkyl, cycloalkyl C₁-C₁₂aryl, substituted alkyl, substituted cycloalkyl and substituted C₁-C₁₂aryl, and
R⁵ and R⁶ are each independently selected from hydrogen, alkyl, C₃₋₆cycloalkyl, aryl or R⁵ and R⁶ taken together with the nitrogen to which they are attached represent a 5 to 6 member saturated ring containing up to one other heteroatom selected from oxygen and nitrogen;
m is 0-6; and
R¹⁰ is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, cycloalkyl, substituted cycloalkyl, aryl, substituted aryl, aryloxy, alkoxy, acyloxy, amino, nitro, cyano, halogen, hydroxy, and alkyl substituted with one or more substituents selected from the group consisting of: alkoxy, acyloxy, aryl, substituted aryl cycloalkyl, substituted cycloalkyl, aryloxy, amino, nitro, cyano, halogen, and hydroxy; and pharmaceutically acceptable salts, hydrates, solvates and esters thereof;
and wherein:
by the term "aryl", unless otherwise defined, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbon atoms is 3 the aromatic ring contains at least two heteroatoms and when the number of carbon atoms is 4 the aromatic ring contains at least one heteroatom;
by the term "C₁-C₁₂aryl", unless otherwise defined, is meant phenyl, naphthalene, 3,4-methylenedioxyphenyl, pyridine, biphenyl, quinoline, pyrimidine, quinazoline, thiophene, furan, pyrrole, pyrazole, imidazole and tetrazole;
by the term "substituted", unless otherwise defined, is meant that the subject chemical moiety has one or more substituents selected from the group consisting of: hydroxyalkyl, alkoxy, acyloxy, alkyl, amino, N-acylamino, hydroxy, -(CH₂)_{g}C(O)OR¹¹, -S(O)ₙR¹², nitro, cyano, halogen, and trifluoromethyl, where g is 0-6, R¹¹ is hydrogen or alkyl, n is 0-2, and R¹² is hydrogen or alkyl;
by the term "alkoxy" is meant -Oalkyl where alkyl is as described herein;
by the term "cycloalkyl", unless otherwise defined, is meant a nonaromatic, unsaturated or saturated, cyclic or polycyclic C₃-C₁₂,
by the term "acyloxy" is meant -OC(O)alkyl where alkyl is as described herein;
by the term ''N-acylamino'' is meant N(H)C(O)alkyl, where alkyl is as described herein;
by the term "aryloxy" is meant -OC₆-C₁₂aryl where C₆-C₁₂aryl is phenyl, naphthyl, 3,4-methylenedioxyphenyl, or biphenyl optionally substituted with one or more substituents selected from the group consisting of: alkyl, hydroxyakyl, alkoxy, trifluoromethyl, acyloxy, amino, N-acylamino, hydroxy, -(CH₂)_{g}C(O)C)R¹¹, -S(O)ₙR¹², nitro, cyano, and halogen, where g is 0-6, R¹¹ is hydrogen or alkyl, n is 0-2 and R¹² is hydrogen or alkyl;
by the term "heteroatom" is meant oxygen, nitrogen or sulfur;
by the term "halogen" as used herein is meant a substituent selected from bromide, iodide, chloride and fluoride; and
by the term "alkyl" and derivatives thereof and in all carbon chains as used herein is meant a linear or branched, saturated or unsaturated hydrocarbon chain, and unless otherwise defined the carbon chain will contain from 1 to 12 carbon atoms;
provided that:
at least one of R, R¹, R² and R³ is: sulfonic acid, -C(O)OR⁴, tetrazole, -CONR⁵R⁶, phosphonic acid or phosphinic acid; where R⁴, R⁵ and R⁶ are as described above;
and provided that:
when R¹ is carboxylic acid; R, R² and R³ are hydrogen; and R⁹ is methyl;
R¹⁰ is not unsubstituted phenyl.

13. A medicament as claimed in claim 12 wherein, in the compound of Formula (I),
R¹ is carboxylic acid or sulfonic acid;
R, R² and R³ are each independently selected from hydrogen, C₁₋₆alkoxy, tetrazole, -CONR⁵R⁶, phosphonic acid, phosphinic acid, C₁₋₆alkyl and halogen;
m is 0; and
R⁹ is C₁₋₆alkyl, C₁₋₆alkoxy, halogen, or C₁-C₁₂aryl; and
R¹⁰ is phenyl substituted with one or more substituents selected from the group consisting of: alkyl, substituted alkyl, alkoxy, trifluoromethyl, halogen and hydroxy.

14. A medicament as claimed in claim 12 wherein, in the compound of Formula (I),
R¹ is carboxylic acid or sulfonic acid;
R, R² and R³ are each independently selected from hydrogen. C₁₋₆alkoxy. C₁₋₆alkyl and halogen;
m is 0; and
R⁹ is C₁₋₆alkyl or C₁₋₆alkoxy; and
R¹⁰ is phenyl substituted with from one to three substituents selected from the group consisting of: alkyl, hydroxy, alkoxy, trifluoromethyl and halogen.

15. A medicament as claimed in claim 12, 13 or 14, wherein, in the compound of Formula (I),
the term "alkyl", including in derivatives thereof, means -CH₃, -CH₂-CH₃, -CH₂-CH₂-CH₃, -CH(CH₃)₂, -C(CH₃)₃, -(CH₂)3-CH₃, -CH₂-CH(CH₃)₂, or -CH(CH₃)-CH₂-CH₃,
and the term "substituted" means that the subject chemical moiety has one substituent selected from the group consisting of: hydroxyalkyl, alkoxy, acyloxy, alkyl, amino, N-acylamino, hydroxy, C(O)OR¹¹, -S(O)ₙR¹², nitro, cyano, halogen, and trifluoromethyl, wherein R¹ is hydrogen or alkyl, n is 0-2. and R¹² is hydrogen or alkyl.

16. A medicament as claimed in claim 12, wherein the compound of Formula (I) is selected from:
3-hydroxy-4-{[5-hydroxy-3-methyl-1-(4-methylphenyl)-1H-pyrazol-4-yl]azo}benzoic acid;
4-{[1-(4-benzyloxyphenyl)-5-hydroxy-3-methyl-1H-pyrazol-4-yl]azo}-3-hydroxybenzoic acid;
4-{[1-(4-chlorophenyl)-5-hydroxy-3-methyl-1H-pyraxol-4-yl]azo}-3-hydroxybenzoic acid;
4-{[1-(3-chlorophenyl)-5-hydroxy-3-methyl-1H-pyrazol-4-yl]azo}-3-hydroxybenzoic acid;
4-{[1-(3,4-dimethylphenyl)-5-hydroxy-3 methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-4-hydroxybenzoic acid;
3-hydroxy-4-{[5-bydroxy-3-methyl-1-(3-methylphenyl)-1H-pyrazol-4-yl]azo}benzoic acid;
3-hydroxy-4-{[5-hydroxy-3-methyl-1-(4-trifluoremethylphenyl)-1H-pyrazol-4-yl]azo}benzoic acid;
3-{[1-(3,4-dimethylphenyl)-5-hydroxy-3-methyl-1H-pyrazol-4-yl]azo}-2-hydroxybenzoic acid;
2-{[1-(3,4-dimethylphenyl)-5-hydroxy-3-methyl-1H-pyrazol-4-yl]azo}-3-hydroxybenzoic acid;
5-chloro-3-{[1-(3,4-dimethylphenyl)-5-hydroxy-3-methyl-1H-pyrazol-4-yl]azo}-2-hydroxybenzenesulfonic acid;
3*-tert-*butyl-4-{[1-(3,4-dimethylphenyl)-5-hydroxy-1H-pyrazol-4-yl]azo}-3-hydroxybenzoic acid;
4-{[1-(3,4-dimethylphenyl)-5-hydroxy-3-phenyl-1H-pyrazol-4-yl]azo}-3-hydroxybenzoic acid;
methyl 3-hydroxy-4-{[5-hydroxy-3-methyl-1-(4-methylphenyl)-1H-pyrazol-4-yl]azo}benzoate; and
4-{[1-(4-*tert*-butylphenyl)-5-hydroxy-3-methyl-1H-pyrazol-4-yl]azo}-3-hydroxybenzoic acid;
and pharmaceutically acceptable salts, hydrates, solvates and esters thereof.

17. A compound selected from
3-hydroxy-4-{[5-hydroxy-3-methyl-1-(4-methylphenyl-1H-pyrazol-4-yl]azo}benzoic acid;
4-{[1-(4-benzyloxyphenyl)-5-hydroxy-3-methyl-1H-pyrazol-4-yl]azo}-3-hydroxybenzoic acid;
4-{[1-(4-chlorophenyl)-5-hydroxy-3-methyl-1H-pyrazol-4-yl]azo}-3-hydroxybenzoic acid;
4-{[1-(3-chlorophenyl)-5-hydroxy-3-methyl-1H-pyrazol-4-yl]azo}-3-hydroxybenzoic acid;
4-{[1-(3,4-dimethylphenyl)-5-hydroxy-3-methyl-1H-pyrazol-4-yl]azo}-3-hydroxybenzoic acid;
3-{[1-(3,4-dimethylphenyl)-5-hydroxy-3-methyl-1H-pyrazol-4-yl]azo}-4. hydroxybenzoic acid;
3-hydroxy-4-{[5-hydroxy-3-methyl-1-(3-methylphenyl)-1H-pyrazol-4-yl]azo}benzoic acid;
3-hydroxy-4-{[5-hydroxy-3-methyl-1-(4-trifluoromethylphenyl)-1H-pyrazol-4-yl]azo}benxoic acid;
3-{[1-(3,4-dimethylphenyl)-5-hydroxy-3-methyl-1H-pyrazol-4-yl]azo}-2-hydroxybenzoic acid;
2-{[1-(3,4-dimethylphenyl)-5-hydroxy-3-methyl-1H-pyrazol-4-yl]azo}-3-hydroxybenzoic acid;
5-chloro-3-{[1-(3,4-dimethylphenyl)-5-hydroxy-3-methyl-1H-pyrazol-4-yl]azo}-2-hydroxybenzenesulfonic acid;
3-*tert*-butyl-4-{[1-(3,4-dimethylphenyl)-5-hydroxy-1H-pyrazol-4-yl]azo}-3-hydroxybenzoic acid;
4-{[1-(3,4-dimethylphenyl)-5-hydroxy-3-phenyl-1H-pyrazol-4-yl]azo}-3-hydroxybenzoic acid;
methyl 3-hydroxy-4-{[5-hydroxy-3-methyl-1-(4-methylphenyl)-1H-pyrazol-4-yl]azo}benzoate; and
4-{[1-(4-*tert*-butylphenyl)-5-hydroxy-3-methyl-1H-pyrazol-4-yl]azo}-3-hydroxybenzoic acid;
and pharmaceutically acceptable salts, hydrates, solvates and esters thereof.

18. A pharmaceutical composition comprising a compound represented by Formula (I), as defined in any of claims 12 to 16, and a pharmaceutical carrier.

19. A pharmaceutical composition for use in administering to and enhancing platelet production in a mammal, including a human, in need of enhanced platelet production,
which composition comprises a therapeutically effective amount of a compound of Formula (I), as defined in any of claims 12 to 16, and a pharmaceutically acceptable carrier.

## Patentansprüche

1. Verwendung einer Verbindung der Formel (II) 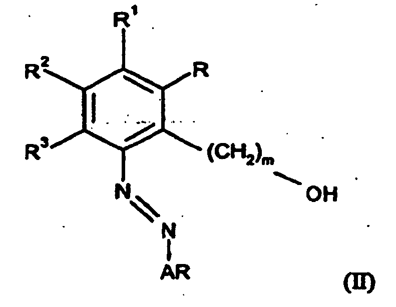
worin:
R, R¹, R² und R³ jeweils unabhängig ausgewählt sind aus Wasserstoff, C₁₋₆-Alkyl, C₁₋₆-Alkoxy, -(CH₂)ₚOR⁴, -C(O)OR⁴, Nitro, Cyano, Halogen, Aryl, -S (O)ₙR⁴, Cycloalkyl, -CONR⁵R⁶, -NR⁵R⁶, Phosphonsäure, Sulfonsäure, Phosphinsäure und -SO₂NR⁵R⁶,
worin
p 0-6 ist;
n 0-2 ist;
R⁴ Wasserstoff, Alkyl, Cycloalkyl, C₁₋₁₂-Aryl, substituiertes Alkyl, substituiertes Cycloalkyl und substituiertes C₁₋₁₂-Aryl ist und
R⁵ und R⁶ jeweils unabhängig aus Wasserstoff, Alkyl, C₃₋₆-Cycloalkyl und Aryl ausgewählt sind oder R⁵ und R⁶ zusammen mit dem Stickstoff, an den sie gebunden sind, einen 5- bis 6-gliedrigen gesättigten Ring darstellen, der bis zu einem anderen Heteroatom enthält, das aus Sauerstoff und Stickstoff ausgewählt ist;
m 0-6 ist; und
AR ein cyclischer oder polycyclischer aromatischer Ring ist, der 3 bis 16 Kohlenstoffatome enthält und gegebenenfalls ein oder mehrere Heteroatome enthält, mit der Maßgabe, daß dann, wenn die Anzahl der Kohlenstoffatome 3 ist, der aromatische Ring wenigstens zwei Heteroatome enthält, und dann, wenn die Anzahl der Kohlenstoffatome 4 ist, der aromatische Ring wenigstens ein Heteroatom enthält, und der gegebenenfalls mit einem oder mehreren Substituenten substituiert ist, die aus der Gruppe ausgewählt sind, die aus folgenden besteht: Alkyl, Aryl, substituiertem Cycloalkyl, substituiertem Aryl, Aryloxy, Hydroxy, Alkoxy, Cycloalkyl, Acyloxy, Amino, N-Acylamino, Nitro, Cyano, Halogen, -C(O)OR⁴, -C(O)NR⁷R⁸, -S(O)₂NR⁷R⁸, -S(O)ₙR⁴ und Alkyl, das mit einem oder mehreren Substituenten substituiert ist, die aus der Gruppe ausgewählt sind, die aus Alkoxy, Acyloxy, Aryl, substituiertem Aryl, Amino, N-Acylamino, Oxo, Hydroxy, Cycloalkyl, substituiertem Cycloalkyl, -C(O)OR⁴, -C(O)NR⁷R⁸, -S(O)₂NR⁷R⁸, -S(O)ₙR⁴, Aryloxy, Nitro, Cyano und Halogen besteht,
worin
R⁴ Wasserstoff, Alkyl, Cycloalkyl, C₁₋₁₂-Aryl, substituiertes Alkyl, substituiertes Cycloalkyl und substituiertes C₁₋₁₂-Aryl ist; und
R⁷ und R⁸ unabhängig Wasserstoff, Cycloalkyl, C₁₋₁₂₋Aryl, substituiertes Cycloalkyl, substituiertes C₁₋₁₂₋Aryl, Alkyl oder Alkyl sind, das mit einem oder mehreren Substituenten substituiert ist, die aus der Gruppe ausgewählt sind, die aus Alkoxy, Acyloxy, Aryloxy, Amino, N-Acylamino, Oxo, Hydroxy, -C(O)OR⁴, -S(O)ₙR⁴, -C(O)NR⁴R⁴, -S(O)₂NR⁴R⁴, Nitro, Cyano, Cycloalkyl, substituiertem Cycloalkyl, Halogen, C₁₋₁₂₋Aryl und substituiertem C₁₋₁₂-Aryl besteht,
oder R⁷ und R⁸ zusammen mit dem Stickstoff, an den sie gebunden sind, einen 5- bis 6-gliedrigen gesättigten Ring darstellen, der bis zu einem anderen Heteroatom enthält, das aus Sauerstoff und Stickstoff ausgewählt ist,
worin R⁴ wie oben beschrieben ist und n 0-2 ist; und
pharmazeutisch akzeptabler Salze, Hydrate, Solvate und Ester davon;
und worin:
mit dem Begriff "Aryl", wenn nicht anders definiert, ein cyclischer oder polycyclischer aromatischer Ring gemeint ist, der 1 bis 14 Kohlenstoffatome enthält und gegebenenfalls 1 bis 5 Heteroatome enthält, mit der Maßgabe, daß dann, wenn die Anzahl der Kohlenstoffatome 1 ist, der aromatische Ring wenigstens 4 Heteroatome enthält, dann, wenn die Anzahl der Kohlenstoffatome 2 ist, der aromatische Ring wenigstens 3 Heteroatome enthält, dann, wenn die Anzahl der Kohlenstoffatome 3 ist, der aromatische Ring wenigstens zwei Heteroatome enthält, und dann, wenn die Anzahl der Kohlenstoffatome 4 ist, der aromatische Ring wenigstens ein Heteroatom enthält;
mit dem Begriff "C₁₋₁₂-Aryl", wenn nicht anders definiert, Phenyl, Naphthalin, 3,4-Methylendioxyphenyl, Pyridin, Biphenyl, Chinolin, Pyrimidin, Chinazolin, Thiophen, Furan, Pyrrol, Pyrazol, Imidazol und Tetrazol gemeint ist;
mit dem Begriff "substituiert", wenn nicht anders definiert, gemeint ist, daß die betroffene chemische Einheit einen oder mehrere Substituenten aufweist, die aus der Gruppe ausgewählt sind, die aus Hydroxyalkyl, Alkoxy, Acyloxy, Alkyl, Amino, N-Acylamino, Hydroxy, -(CH₂)_{g}C(O)OR¹¹, -S(O)ₙR¹², Nitro, Cyano, Halogen und Trifluormethyl besteht, worin g 0-6 ist, R¹¹ Wasserstoff oder Alkyl ist, n 0-2 ist und R¹² Wasserstoff oder Alkyl ist;
mit dem Begriff "Alkoxy" -OAlkyl gemeint ist, worin Alkyl wie hier beschrieben ist;
mit dem Begriff "Cycloalkyl", wenn nicht anders definiert, ein nicht-aromatisches, ungesättigtes oder gesättigtes, cyclisches oder polycyclisches C₃₋₁₂ gemeint ist;
mit dem Begriff "Acyloxy" -OC(O)-Alkyl gemeint ist, worin Alkyl wie hier beschrieben ist;
mit dem.Begriff "N-Acylamino" -N(H)C(O)-Alkyl gemeint ist, worin Alkyl wie hier beschrieben ist;
mit dem Begriff "Aryloxy" -OC₆₋₁₂-Aryl gemeint ist, worin C₆₋₁₂-Aryl Phenyl, Naphthyl, 3,4-Methylendioxyphenyl oder Biphenyl ist, gegebenenfalls substituiert mit einem oder mehreren Substituenten, die aus der Gruppe ausgewählt sind, die aus Alkyl, Hydroxyalkyl, Alkoxy, Trifluormethyl, Acyloxy, Amino, N-Acylamino, Hydroxy, -(CH₂)_{g}C(O)OR¹¹, -S(O)ₙR¹², Nitro, Cyano und Halogen besteht, worin g 0-6 ist, R¹¹ Wasserstoff oder Alkyl ist, n 0-2 ist und R¹² Wasserstoff oder Alkyl ist;
mit dem Begriff "Heteroatom" Sauerstoff, Stickstoff oder Schwefel gemeint ist;
mit dem Begriff "Halogen" ein Substituent gemeint ist, der aus Bromid, Iodid, Chlorid und Fluorid ausgewählt ist; und
mit dem Begriff "Alkyl" und Derivaten davon und in allen Kohlenstoffketten wie hier verwendet eine lineare oder verzweigte, gesättigte oder ungesättigte Kohlenwasserstoffkette gemeint ist und die Kohlenstoffkette, wenn nicht anders definiert, 1 bis 12 Kohlenstoffatome enthalten wird;
in der Herstellung eines Medikaments zur Verwendung in der Behandlung von Thrombozytopenie.

2. Verwendung gemäß Anspruch 1, worin in der Verbindung der Formel (II)
R¹ Carbonsäure oder Sulfonsäure ist;
R, R² und R³ jeweils unabhängig ausgewählt sind aus Wasserstoff, C₁₋₆-Alkoxy, Tetrazol, -CONR⁵R⁶, Phosphonsäure, Phosphinsäure, C₁₋₆-Alkyl und Halogen;
m 0 ist; und
AR ein cyclischer oder polycyclischer aromatischer Ring ist, der 3 bis 14 Kohlenstoffatome enthält und gegebenenfalls ein oder mehrere Heteroatome enthält, mit der Maßgabe, daß dann, wenn die Anzahl der Kohlenstoffatome 3 ist, der aromatische Ring wenigstens zwei Heteroatome enthält, und dann, wenn die Anzahl der Kohlenstoffatome 4 ist, der aromatische Ring wenigstens ein Heteroatom enthält, und der gegebenenfalls mit einem oder mehreren Substituenten substituiert ist, die aus der Gruppe ausgewählt sind, die aus Alkyl, C₁₋₁₂₋Aryl, substituiertem C₁₋₁₂-Aryl, Aryloxy, Hydroxy, Alkoxy, Amino und Halogen besteht.

3. Verwendung gemäß Anspruch 1, worin in der Verbindung der Formel (II)
R¹ Carbonsäure oder Sulfonsäure ist;
R, R² und R³ jeweils unabhängig ausgewählt sind aus Wasserstoff, C₁₋₆-Alkoxy, C₁₋₆-Alkyl und Halogen;
m 0 ist; und
AR aus Naphthalin, Phenyl und Pyrazol ausgewählt ist und gegebenenfalls mit einem bis drei Substituenten substituiert ist, die aus der Gruppe ausgewählt sind, die aus Alkyl, C₁₋₁₂-Aryl, substituiertem C₁₋₁₂-Aryl, Hydroxy, Alkoxy und Halogen besteht.

4. Verwendung gemäß Anspruch 1, worin in der Verbindung der Formel (II)
R¹ Carbonsäure oder Sulfonsäure ist;
R, R² und R³ jeweils unabhängig ausgewählt sind aus Wasserstoff, C₁₋₆-Alkoxy, C₁₋₆-Alkyl und Halogen;
m 0 ist; und
AR Pyrazol ist, das gegebenenfalls mit einem bis drei Substituenten substituiert ist, die aus der Gruppe ausgewählt sind, die aus Alkyl, C₁₋₁₂-Aryl, substituiertem C₁₋₁₂-Aryl, Hydroxy, Alkoxy und Halogen besteht.

5. Verwendung gemäß Anspruch 1, worin die Verbindung ausgewählt ist aus
3-Hydroxy-4-{[5-hydroxy-3-methyl-1-(4-methylphenyl)-1H-pyrazol-4-yl]azo}benzoesäure;
3-Hydroxy-4-[(5-hydroxy-3-methyl-1-phenyl-1H-pyrazol-4-yl)azo]benzoesäure;
4-{[1-(4-Benzyloxyphenyl)-5-hydroxy-3-methyl-1H-pyrazol-4-yl]azo}-3-hydroxybenzoesäure;
4-{[1-(4-Chlorphenyl)-5-hydroxy-3-methyl-1H-pyrazol-4-yl]azo}-3-hydroxybenzoesäure;
4-{[1-(3-Chlorphenyl)-5-hydroxy-3-methyl-1H-pyrazol-4-yl]azo}-3-hydroxybenzoesäure;
4-{[1-(3,4-Dimethylphenyl)-5-hydroxy-3-methyl-1H-pyrazol-4-yl]azo}-3-hydroxybenzoesäure;
3-{[1-(3,4-Dimethylphenyl)-5-hydroxy-3-methyl-1H-pyrazol-4-yl]azo}-4-hydroxybenzoesäure;
3-Hydroxy-4-{[5-hydroxy-3-methyl-1-(3-methylphenyl)-1H-pyrazol-4-yl]azo}benzoesäure;
3-Hydroxy-4-{[5-hydroxy-3-methyl-1-(4-trifluormethylphenyl)-1H-pyrazol-4-yl]azo}benzoesäure;
3-{[1-(3,4-Dimethylphenyl)-5-hydroxy-3-methyl-1H-pyrazol-4-yl]azo}-2-hydroxybenzoesäure;
2-{[1-(3,4-Dimethylphenyl)-5-hydroxy-3-methyl-1H-pyrazol-4-yl]azo}-3-hydroxybenzoesäure;
5-Chlor-3-{[1-(3,4-dimethylphenyl)-5-hydroxy-3-methyl-1H-pyrazol-4-yl]azo}-2-hydroxybenzolsulfonsäure;
3-tert-Butyl-4-{[1-(3,4-dimethylphenyl)-5-hydroxy-1H-pyrazol-4-yl]azo}-3-hydroxybenzoesäure;
4-{[1-(3,4-Dimethylphenyl)-5-hydroxy-3-phenyl-1H-pyrazol-4-yl]azo}-3-hydroxybenzoesäure;
Methyl-3-hydroxy-4-{[5-hydroxy-3-methyl-1-(4-methylphenyl)-1H-pyrazol-4-yl]azo}benzoat und
4-{[1-(4-tert-Butylphenyl)-5-hydroxy-3-methyl-1H-pyrazol-4-yl]azo}-3-hydroxybenzoesäure;
und pharmazeutisch akzeptablen Salzen, Hydraten, Solvaten und Estern davon.

6. Verwendung einer Verbindung der Formel (II) wie in einem der vorhergehenden Ansprüche definiert in der Herstellung eines Medikaments,
worin das Medikament zur Verwendung in der Verabreichung einer therapeutisch wirksamen Menge der Verbindung an ein Säugetier und in der Steigerung der Blutplättchenproduktion im Säugetier ist,
und worin das Säugetier, einschließlich Mensch, eine gesteigerte Blutplättchenproduktion benötigt.

7. Verwendung einer Verbindung der Formel (II) wie in einem der vorhergehenden Ansprüche beschrieben in der Herstellung eines Medikaments zur Verwendung in.der Verabreichung einer wirksamen Menge der Verbindung an einen und zur Förderung ("Agonisieren") des TPO-Rezeptors in einem Patienten.

8. Pharmazeutische Zusammensetzung zur Verwendung in der Behandlung von Thrombozytopenie, die eine Verbindung der Formel (II) wie in einem der vorhergehenden Ansprüche definiert und einen pharmazeutisch akzeptablen Träger umfaßt.

9. Pharmazeutische Zusammensetzung zur Verwendung in der Verabreichung an ein und Steigerung der Blutplättchenproduktion in einem Säugetier, einschließlich Mensch, das eine gesteigerte Blutplättchenproduktion benötigt,
wobei die Zusammensetzung eine therapeutisch wirksame Menge einer Verbindung der Formel (II) wie in einem der vorhergehenden Ansprüche beschrieben und einen pharmazeutisch akzeptablen Träger umfaßt.

10. Verwendung oder Zusammensetzung gemäß einem der vorhergehenden Ansprüche, worin das Medikament oder die Zusammensetzung zur oralen Verabreichung ist.

11. Verwendung oder Zusammensetzung gemäß einem der vorhergehenden Ansprüche, worin das Medikament oder die Zusammensetzung zur parenteralen Verabreichung ist.

12. Medikament zur Verwendung in der Therapie, hergestellt unter Verwendung einer und umfassend eine Verbindung, die durch die folgende Formel (I) dargestellt wird: 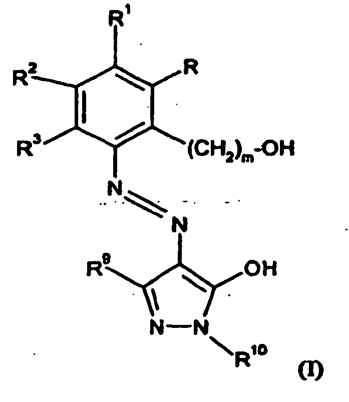
worin:
R, R¹, R², R³ und R⁹ jeweils unabhängig ausgewählt sind aus Wasserstoff, C₁₋₆-Alkyl, C₁₋₆-Alkoxy, -(CH₂)ₚOR⁴, -C(O)OR⁴, Nitro, Cyano, Halogen, Aryl, -S(O)ₙR⁴, Cycloalkyl, -CONR⁵R⁶, Phosphonsäure, Sulfonsäure, Phosphinsäure und -SO₂NR⁵R⁶,
worin
p 0-6 ist;
n 0-2 ist;
R⁴ Wasserstoff, Alkyl, Cycloalkyl, C₁₋₁₂-Aryl, substituiertes Alkyl, substituiertes Cycloalkyl und substituiertes C₁₋₁₂-Aryl ist und
R⁵ und R⁶ jeweils unabhängig aus Wasserstoff, Alkyl, C₃₋₆-Cycloalkyl und Aryl ausgewählt sind oder R⁵ und R⁶ zusammen mit dem Stickstoff, an den sie gebunden sind, einen 5- bis 6-gliedrigen gesättigten Ring darstellen, der bis zu einem anderen Heteroatom enthält, das aus Sauerstoff und Stickstoff ausgewählt ist;
m 0-6 ist; und
R¹⁰ ein cyclischer oder polycyclischer aromatischer Ring ist, der 3 bis 16 Kohlenstoffatome enthält und gegebenenfalls ein oder mehrere Heteroatome enthält, mit der Maßgabe, daß dann, wenn die Anzahl der Kohlenstoffe 3 ist, der aromatische Ring wenigstens zwei Heteroatome enthält, und dann, wenn die Anzahl der Kohlenstoffatome 4 ist, der aromatische Ring wenigstens ein Heteroatom enthält, und der gegebenenfalls mit einem oder mehreren Substituenten substituiert ist, die aus der Gruppe ausgewählt sind, die aus folgenden besteht: Alkyl, Cycloalkyl, substituiertem Cycloalkyl, Aryl, substituiertem Aryl, Aryloxy, Alkoxy, Acyloxy, Amino, Nitro, Cyano, Halogen, Hydroxy und Alkyl, das mit einem oder mehreren Substituenten substituiert ist, die aus der Gruppe ausgewählt sind, die aus Alkoxy, Acyloxy, Aryl, substituiertem Aryl, Cycloalkyl, substituiertem Cycloalkyl, Aryloxy, Amino, Nitro, Cyano, Halogen und Hydroxy besteht; und
pharmazeutisch akzeptable Salze, Hydrate, Solvate und Ester davon; und worin:
mit dem Begriff "Aryl", wenn nicht anders definiert, ein cyclischer oder polycyclischer aromatischer Ring gemeint ist, der 1 bis 14 Kohlenstoffatome enthält und gegebenenfalls 1 bis 5 Heteroatome enthält, mit der Maßgabe, daß dann, wenn die Anzahl der Kohlenstoffatome 1 ist, der aromatische Ring wenigstens 4 Heteroatome enthält, dann, wenn die Anzahl der Kohlenstoffatome 2 ist, der aromatische Ring wenigstens 3 Heteroatome enthält, dann, wenn die Anzahl der Kohlenstoffatome 3 ist, der aromatische Ring wenigstens zwei Heteroatome enthält, und dann, wenn die Anzahl der Kohlenstoffatome 4 ist, der aromatische Ring wenigstens ein Heteroatom enthält;
mit dem Begriff "C₁₋₁₂-Aryl", wenn nicht anders definiert, Phenyl, Naphthalin, 3,4-Methylendioxyphenyl, Pyridin, Biphenyl, Chinolin, Pyrimidin, Chinazolin, Thiophen, Furan, Pyrrol, Pyrazol, Imidazol und Tetrazol gemeint ist;
mit dem Begriff "substituiert", wenn nicht anders definiert, gemeint ist, daß die betroffene chemische Einheit einen oder mehrere Substituenten aufweist, die aus der Gruppe ausgewählt sind, die aus Hydroxyalkyl, Alkoxy, Acyloxy, Alkyl, Amino, N-Acylamino, Hydroxy, -(CH₂)_{g}C(O)OR¹¹, -S(O)ₙR¹², Nitro, Cyano, Halogen und Trifluormethyl besteht, worin g 0-6 ist, R¹¹ Wasserstoff oder Alkyl ist, n 0-2 ist und R¹² Wasserstoff oder Alkyl ist;
mit dem Begriff "Alkoxy" -OAlkyl gemeint ist;
mit dem Begriff "Cycloalkyl", wenn nicht anders definiert, ein nicht-aromatisches, ungesättigtes oder gesättigtes, cyclisches oder polycyclisches C₃₋₁₂ gemeint ist;
mit dem Begriff "Acyloxy" -OC(O)-Alkyl gemeint ist, worin Alkyl wie hier beschrieben ist;
mit dem Begriff "N-Acylamino" -N(H)C(O)-Alkyl gemeint ist, worin Alkyl wie hier beschrieben ist;
mit dem Begriff "Aryloxy" -OC₆₋₁₂-Aryl gemeint ist, worin C₆₋₁₂-Aryl Phenyl, Naphthyl, 3,4-Methylendioxyphenyl oder Biphenyl ist, gegebenenfalls substituiert mit einem oder mehreren Substituenten, die aus der Gruppe ausgewählt sind, die aus Alkyl, Hydroxyalkyl, Alkoxy, Trifluormethyl, Acyloxy, Amino, N-Acylamino, Hydroxy, -(CH₂)_{g}C(O)OR¹¹, -S(O)ₙR¹², Nitro, Cyano und Halogen besteht, worin g 0-6 ist, R¹¹ Wasserstoff oder Alkyl ist, n 0-2 ist und R¹² Wasserstoff oder Alkyl ist;
mit dem Begriff. "Heteroatom" Sauerstoff, Stickstoff oder Schwefel gemeint ist;
mit dem Begriff "Halogen" ein Substituent gemeint ist, der aus Bromid, Iodid, Chlorid und Fluorid ausgewählt ist; und
mit dem Begriff "Alkyl" und Derivaten davon und in allen Kohlenstoffketten wie hier verwendet eine lineare oder verzweigte, gesättigte oder ungesättigte Kohlenwasserstoffkette gemeint ist und die Kohlenstoffkette, wenn nicht anders definiert, 1 bis 12 Kohlenstoffatome enthalten wird;
mit der Maßgabe daß:
wenigstens eines aus R, R¹, R² und R³ Sulfonsäure, -C(O)OR⁴, Tetrazol, -CONR⁵R⁶, Phosphonsäure oder Phosphinsäure ist; worin R⁴, R⁵ und R⁶ wie oben beschrieben sind;
und mit der Maßgabe daß:
wenn R¹ Carbonsäure ist; R, R² und R³ Wasserstoff sind; und R⁹ Methyl ist; R¹⁰ nicht unsubstituiertes Phenyl ist.

13. Medikament gemäß Anspruch 12, worin in der Verbindung der Formel (I)
R¹ Carbonsäure oder Sulfonsäure ist;
R, R² und R³ jeweils unabhängig ausgewählt sind aus ' Wasserstoff, C₁₋₆-Alkoxy, Tetrazol, -CONR⁵R⁶, Phosphonsäure, Phosphinsäure, C₁₋₆-Alkyl und Halogen;
m 0 ist; und
R⁹ C₁₋₆-Alkyl, C₁₋₆-Alkoxy, Halogen oder C₁₋₁₂-Aryl ist; und
R¹⁰ Phenyl ist, das mit einem oder mehreren Substituenten substituiert ist, die aus der Gruppe ausgewählt sind, die aus Alkyl, substituiertem Alkyl, Alkoxy, Trifluormethyl, Halogen und Hydroxy besteht.

14. Medikament gemäß Anspruch 12, worin in der Verbindung der Formel (I)
R¹ Carbonsäure oder Sulfonsäure ist;
R, R² und-R³ jeweils unabhängig ausgewählt sind aus Wasserstoff, C₁₋₆-Alkoxy, C₁₋₆-Alkyl und Halogen;
m 0 ist; und
R⁹ C₁₋₆-Alkyl oder C₁₋₆-Alkoxy ist; und
R¹⁰ Phenyl ist, das mit einem bis drei Substituenten substituiert ist, die aus der.Gruppe ausgewählt sind, die aus Alkyl, Hydroxy, Alkoxy, Trifluormethyl und Halogen besteht.

15. Medikament gemäß Anspruch 12, 13 oder 14, worin in der Verbindung der Formel (I)
der Begriff "Alkyl", einschließlich der Derivate davon, -CH₃, -CH₂-CH₃, -CH₂-CH₂-CH₃, -CH(CH₃)₂, -C(CH₃)₃, -(CH₂)₃-CH₃, -CH₂-CH(CH₃)₂ oder -CH(CH₃)-CH₂-CH₃ bedeutet,
und der Begriff "substituiert" bedeutet, daß die betroffene chemische Einheit einen Substituenten aufweist, der aus der Gruppe ausgewählt ist, die aus Hydroxyalkyl, Alkoxy, Acyloxy, Alkyl, Amino, N-Acylamino, Hydroxy, C(O)OR¹¹, -S(O)ₙR¹², Nitro, Cyano, Halogen und Trifluormethyl besteht, worin R¹¹ Wasserstoff oder Alkyl ist, n 0-2 ist und R¹² Wasserstoff oder Alkyl ist.

16. Medikament gemäß Anspruch 12, worin die Verbindung der Formel (I) ausgewählt ist aus:
3-Hydroxy-4-{[5-hydroxy-3-methyl-1-(4-methylphenyl)-1H-pyrazol-4-yl]azo}benzoesäure;
4-{[1-(4-Benzyloxyphenyl)-5-hydroxy-3-methyl-1H-pyrazol-4-yl]azo}-3-hydroxybenzoesäure;
4-{[1-(4-Chlorphenyl)-5-hydroxy-3-methyl-1H-pyrazol-4-yl]azo}-3-hydroxybenzoesäure;
4-{[1-(3-Chlorphenyl)-5-hydroxy-3-methyl-1H-pyrazol-4-yl]azo}-3-hydroxybenzoesäure;
4-{[1-(3,4-Dimethylphenyl)-5-hydroxy-3-methyl-1H-pyrazol-4-yl]azo}-3-hydroxybenzoesäure;
3-{[1-(3,4-Dimethylphenyl)-5-hydroxy-3-methyl-1H-pyrazol-4-yl]azo}-4-hydroxybenzoesäure;
3-Hydroxy-4-{[5-hydroxy-3-methyl-1-(3-methylphenyl)-1H-pyrazol-4-yl]azo}benzoesäure;
3-Hydroxy-4-{[5-hydroxy-3-methyl-1-(4-trifluormethylphenyl)-1H-pyrazol-4-yl]azo}benzoesäure;
3-{[1-(3,4-Dimethylphenyl)-5-hydroxy-3-methyl-1H-pyrazol-4-yl]azo}-2-hydroxybenzoesäure;
2-{[1-(3,4-Dimethylphenyl)-5-hydroxy-3-methyl-1H-pyrazol-4-yl]azo}-3-hydroxybenzoesäure;
5-Chlor-3-{[1-(3,4-dimethylphenyl)-5-hydroxy-3-methyl-1H-pyrazol-4-yl]azo}-2-hydroxybenzolsulfonsäure;
3-tert-Butyl-4-{[1-(3,4-dimethylphenyl)-5-hydroxy-1H-pyrazol-4-yl]azo}-3-hydroxybenzoesäure;
4-{[1-(3,4-Dimethylphenyl)-5-hydroxy-3-phenyl-1H-pyrazol-4-yl]azo}-3-hydroxybenzoesäure;
Methyl-3-hydroxy-4-{[5-hydroxy-3-methyl-1-(4-methylphenyl)-1H-pyrazol-4-yl]azo}benzoat und 4-{[1-(4-tert-Butylphenyl)-5-hydroxy-3-methyl-1H-pyrazol-4-yl]azo}-3-hydroxybenzoesäure;
und pharmazeutisch akzeptablen Salzen, Hydraten, Solvaten und Estern davon.

17. Verbindung, die ausgewählt ist aus
3-Hydroxy-4-{[5-hydroxy-3-methyl-1-(4-methylphenyl)-1H-pyrazol-4-yl]azo}benzoesäure;
4-{[1-(4-Benzyloxyphenyl)-5-hydroxy-3-methyl-1H-pyrazol-4-yl]azo}-3-hydroxybenzoesäure;
4-{[1-(4-Chlorphenyl)-5-hydroxy-3-methyl-1H-pyrazol-4-yl]azo}-3-hydroxybenzoesäure;
4-{[1-(3-Chlorphenyl)-5-hydroxy-3-methyl-1H-pyrazol-4-yl]azo}-3-hydroxybenzoesäure;
4-{[1-(3,4-Dimethylphenyl)-5-hydroxy-3-methyl-1H-pyrazol-4-yl]azo}-3-hydroxybenzoesäure;
3-{[1-(3,4-Dimethylphenyl)-5-hydroxy-3-methyl-1H-pyrazol-4-yl]azo}-4-hydroxybenzoesäure;
3-Hydroxy-4-{[5-hydroxy-3-methyl-1-(3-methylphenyl)-1H-pyrazol-4-yl]azo}benzoesäure;
3-Hydroxy-4-{[5-hydroxy-3-methyl-1-(4-trifluormethylphenyl)-1H-pyrazol-4-yl]azo}benzoesäure;
3-{[1-(3,4-Dimethylphenyl)-5-hydroxy-3-methyl-1H-pyrazol-4-yl]azo)-2-hydroxybenzoesäure;
2-{[1-(3,4-Dimethylphenyl)-5-hydroxy-3-methyl-1H-pyrazol-4-yl]azo}-3-hydroxybenzoesäure;
5-Chlor-3-{[1-(3,4-dimethylphenyl)-5-hydroxy-3-methyl-1H-pyrazol-4-yl]azo}-2-hydroxybenzolsulfonsäure;
3-tert-Butyl-4-{[1-(3,4-dimethylphenyl}-5-hydroxy-1H-pyrazol-4-yl]azo}-3-hydroxybenzoesäure;
4-{[1-(3,4-Dimethylphenyl)-5-hydroxy-3-phenyl-1H-pyrazol-4-yl]azo}-3-hydroxybenzoesäure;
Methyl-3-hydroxy-4-{[5-hydroxy-3-methyl-1-(4-methylphenyl)-1H-pyrazol-4-yl]azo}benzoat und
4-{[1-(4-tert-Butylphenyl)-5-hydroxy-3-methyl-1H-pyrazol-4-yl]azo}-3-hydroxybenzoesäure;
und pharmazeutisch akzeptablen Salzen, Hydraten, Solvaten und Estern davon.

18. Pharmazeutische Zusammensetzung, die eine durch Formel (I) wie in einem der Ansprüche 12 bis 16 definiert dargestellte Verbindung und einen pharmazeutischen Träger umfaßt.

19. Pharmazeutische Zusammensetzung zur Verwendung in der Verabreichung an ein und Steigerung der Blutplättchenproduktion in einem Säugetier, einschließlich Mensch, das eine gesteigerte Blutplättchenproduktion benötigt,
wobei die Zusammensetzung eine therapeutisch wirksame Menge einer Verbindung der Formel (I) wie in einem der Ansprüche 12 bis 16 definiert und einen pharmazeutisch akzeptablen Träger umfaßt.

## Revendications

1. Utilisation d'un composé de Formule (II) 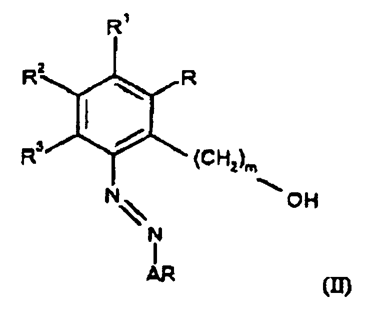
dans laquelle :
- R, R¹, R² et R³ sont chacun indépendamment choisis parmi l'hydrogène, les groupes alkyle C₁₋₆, alcoxy C₁₋₆, -(CH₂)ₚOR⁴, -C(O)OR⁴, nitro, cyano, halogène, aryle, -S(O)ₙR⁴, cycloalkyle, -CONR⁵R⁶, -NR⁵R⁶, acide phosphonique, acide sulfonique, acide phosphinique et -SO₂NR⁵R⁶ ;
où
- p est de 0 à 6 ;
- n est de 0 à 2 ;
- R⁴ représente l'hydrogène, un groupe alkyle, cycloalkyle, aryle C₁-C₁₂, alkyle substitué, cycloalkyle substitué et aryle C₁-C₁₂ substitué, et
- R⁵ et R⁶ sont choisis chacun indépendamment parmi l'hydrogène, les groupes alkyle, cycloalkyle C₃₋₆, aryle, ou bien R⁵ et R⁶ pris en association avec l'azote auquel ils sont attachés, représentent un cycle saturé de 5 à 6 atomes contenant jusqu'à un autre hétéroatome choisi parmi l'oxygène et l'azote ;
- m est de 0 à 6 ; et
- AR représente un cycle aromatique cyclique ou polycyclique contenant 3 à 16 atomes de carbone et contenant éventuellement un ou plusieurs hétéroatomes, à la condition que lorsque le nombre d'atomes de carbone est égal à 3, le cycle aromatique contienne au moins deux hétéroatomes et que lorsque le nombre d'atomes de carbone est égal à 4, le cycle aromatique contienne au moins un hétéroatome, et éventuellement substitué par un ou plusieurs substituants choisis au sein du groupe consistant en des groupes alkyle, aryle, cycloalkyle substitué, aryle substitué, aryloxy, hydroxy, alcoxy, cycloalkyle, acyloxy, amino, N-acylamino, nitro, cyano, halogène, -C(O)OR⁴, -C(O)NR⁷R⁸, -S(O)₂NR⁷R⁸, -S(O)ₙR⁴ et alkyle substitué par un ou plusieurs substituants choisis au sein du groupe consistant en des groupes alcoxy, acyloxy, aryle, aryle substitué, amino, N-acylamino, oxo, hydroxy, cycloalkyle, cycloalkyle substitué, -C(O)OR⁴, -C(O)NR⁷R⁸, -S(O)²NR⁷R⁸, -S(O)ₙR⁴, aryloxy, nitro, cyano et halogène,
où
R⁴ représente l'hydrogène, un groupe alkyle, cycloalkyle, aryle C₁-C₁₂, alkyle substitué, cycloalkyle substitué et aryle C₁-C₁₂ substitué ; et
R⁷ et R⁸ représentent indépendamment l'hydrogène, un groupe cycloalkyle, aryle C₁-C₁₂, cycloalkyle substitué, aryle C₁-C₁₂ substitué, alkyle ou alkyle substitué par un ou plusieurs substituants choisis au sein du groupe consistant en des groupes alcoxy, acyloxy, aryloxy, amino, N-acylamino, oxo, hydroxy, -C(O)OR⁴, -S(O)ₙR⁴ , -C(O)NR⁴R⁴, -S(O)₂NR⁴R⁴, nitro, cyano, cycloalkyle, cycloalkyle substitué, halogène, aryle C₁-C₁₂, et aryle C₁-C₁₂ substitué,
ou R⁷ et R⁸ pris en association avec l'azote auquel ils sont attachés représentent un cycle saturé de 5 à 6 atomes contenant jusqu'à un autre hétéroatome choisi parmi l'oxygène et l'azote,
où R⁴ est tel que décrit plus haut et n est de 0 à 2 ; et
ses sels, hydrates, solvates et esters pharmaceutiquement acceptables ;
et dans laquelle
par le terme « aryle », sauf mention contraire, il est désigné un cycle aromatique cyclique ou polycyclique contenant de 1 à 14 atomes de carbone et contenant éventuellement un à cinq hétéroatomes, à la condition que lorsque le nombre d'atomes de carbone est de 1, le cycle aromatique contienne au moins quatre hétéroatomes, que lorsque le nombre d'atomes de carbone est de 2, le cycle aromatique contienne au moins trois hétéroatomes, que lorsque le nombre d'atomes de carbone est de 3, le cycle aromatique contienne au moins deux hétéroatomes et que lorsque le nombre d'atomes de carbone est de 4, le cycle aromatique contienne au moins un hétéroatome ;
par le terme « aryle C₁-C₁₂ », sauf mention contraire, il est désigné le phényle, le naphtalène, le 3,4-méthylènedioxyphényle, la pyridine, le biphényle, la quinoléine, la pyrimidine, la quinazoline, le thiophène, le furanne, le pyrrole, le pyrazole, l'imidazole et le tétrazole :
par le terme « substitué », sauf mention contraire, il est signifié que l'entité chimique concernée a un ou plusieurs substituants choisis au sein du groupe consistant en des groupes hydroxyalkyle, alcoxy, acyloxy, alkyle, amino, N-acylamino, hydroxy, -(CH₂)_{g}C(O)OR¹¹, -S(O)ₙR¹², nitro, cyano, halogène et trifluorométhyle, où g est de 0 à 6, R¹¹ représente l'hydrogène ou un groupe alkyle, n est de 0 à 2 et R¹² représente l'hydrogène ou un groupe alkyle ;
par le terme « alcoxy » il est désigné un groupe -O-alkyle dans lequel le groupe alkyle est tel que décrit ici ;
par le terme « cycloalkyle », sauf mention contraire, il est désigné une entité non aromatique C₃-C₁₂, saturée ou insaturée, cyclique ou polycyclique ;
par le terme « acyloxy », il est désigné un groupe -OC(O)alkyle dans lequel le groupe alkyle est tel que décrit ici ;
par le terme « N-acylamino », il est désigné un groupe -N(H)C(O)alkyle dans lequel le groupe alkyle est tel que décrit ici ;
par le terme « aryloxy », il est désigné un groupe -O-aryle C₆-C₁₂ dans lequel aryle C₆-C₁₂ représente le groupe phényle, naphtyle, 3,4-méthylènedioxyphényle ou biphényle éventuellement substitué par un ou plusieurs substituants choisis au sein du groupe consistant en des groupes alkyle, hydroxyalkyle, alcoxy, trifluorométhyle, acyloxy, amino, N-acylamino, hydroxy, -(CH₂)_{g}C(O)OR¹¹, -S(O)ₙR¹², nitro, cyano et halogène, où g est de 0 à 6, R¹¹ représente l'hydrogène ou un groupe alkyle, n est de 0 à 2 et R¹² représente l'hydrogène ou un groupe alkyle ;
par le terme « hétéroatome », il est désigné l'oxygène, l'azote ou le soufre ;
par le terme « halogène » tel qu'il est employé ici, il est désigné un substituant choisi parmi les groupes bromure, iodure, chlorure et fluorure ; et
par le terme « alkyle » et ses dérivés et dans toutes les chaînes carbonées, tel qu'il est employé ici, il est désigné une chaîne hydrocarbonée linéaire ou ramifiée, saturée ou insaturée, et sauf mention contraire, la chaîne carbonée contiendra de 1 à 12 atomes de carbone ;
dans la fabrication d'un médicament pour l'utilisation dans le traitement de la thrombocytopénie.

2. Utilisation selon la revendication 1, dans laquelle, dans le composé de Formule (II)
R¹ représente un acide carboxylique ou un acide sulfonique ;
R, R² et R³ sont chacun choisis indépendamment parmi l'hydrogène, les groupes alcoxy C₁₋₆, tétrazole, -CONR⁵R⁶, acide phosphonique, acide phosphinique, alkyle C₁₋₆, et halogène ;
m est égal à 0 ; et
AR représente un cycle aromatique cyclique ou polycyclique contenant 3 à 14 atomes de carbone, contenant éventuellement un ou plusieurs hétéroatomes, à la condition que lorsque le nombre d'atomes de carbone est égal à 3, le cycle aromatique contienne au moins deux hétéroatomes et que lorsque le nombre d'atomes de carbone est égal à 4, le cycle aromatique contienne au moins un hétéroatome, et éventuellement substitué par un ou plusieurs substituants choisis au sein du groupe consistant en des groupes alkyle, aryle C₁-C₁₂, aryle C₁-C₁₂ substitué, aryloxy, hydroxy, alcoxy, amino et halogène.

3. Utilisation selon la revendication 1, dans laquelle, dans le composé de Formule (II)
R¹ représente un acide carboxylique ou un acide sulfonique,
R, R² et R³ sont chacun choisis indépendamment parmi l'hydrogène, les groupes alcoxy C₁₋₆, alkyle C₁₋₆ et halogène ;
m et égal à 0 ; et
AR est choisi parmi les groupes naphtalène, phényle et pyrazole et il est éventuellement substitué par un à trois substituants choisis parmi les groupes alkyle, aryle C₁-C₁₂, aryle C₁-C₁₂ substitué, hydroxy, alcoxy et halogène.

4. Utilisation selon la revendication 1, dans laquelle, dans le composé de Formule (II)
R¹ représente un acide carboxylique ou un acide sulfonique ;
R, R² et R³ dont choisis chacun indépendamment parmi l'hydrogène, les groupes alcoxy C₁₋₆, alkyle C₁₋₆ et halogène ;
m est égal à 0 ; et
AR représente un groupe pyrazole éventuellement substitué par un à trois substituants choisis au sein du groupe consistant en des groupes alkyle, aryle C₁-C₁₂, aryle C₁-C₁₂ substitué, hydroxy, alcoxy et halogène.

5. Utilisation selon la revendication 1, dans laquelle le composé est choisi parmi :
- l'acide 3-hydroxy-4-{[5-hydroxy-3-méthyl-1-(4-méthylphényl)-1H-pyrazol-4-yl]azo}benzoïque ;
- l'acide 3-hydroxy-4-[5-hydroxy-3-méthyl-1-phényl-1H-pyrazol-4-yl)azo]benzoïque ;
- l'acide 4-{[1-(4-benzyloxyphényl)-5-hydroxy-3-méthyl-1H-pyrazol-4-yl]azo}-3-hydroxybenzoïque ;
- l'acide 4-{[1-(4-chlorophényl)-5-hydroxy-3-méthyl-1H-pyrazol-4-yl]azo}-3-hydroxybenzoïque ;
- l'acide 4-{[1-(3-chlorophényl)-5-hydroxy-3-méthyl-1H-pyrazol-4-yl]azo}-3-hydroxybenzoïque ;
- l'acide 4-{[1-(3,4-diméthylphényl)-5-hydroxy-3-méthyl-1H-pyrazol-4-yl]azo}-3-hydroxybenzoïque ;
- l'acide 3-{[1-(3,4-diméthylphényl)-5-hydroxy-3-méthyl-1H-pyrazol-4-yl]azo}-4-hydroxybenzoïque ;
- l'acide 3-hydroxy-4-{[5-hydroxy-3-méthyl-1-(3-méthylphényl)-1H-pyrazol-4-yl]azo}benzoïque ;
- l'acide 3-hydroxy-4-{[5-hydroxy-3-méthyl-1-(4-trifluorométhylphényl)-1H-pyrazol-4-yl]azo}benzoïque ;
- l'acide 3-{[1-(3,4-diméthylphényl)-5-hydroxy-3-méthyl-1H-pyrazol-4-yl]azo}-2-hydroxybenzoïque ;
- l'acide 2-{[1-(3,4-diméthylphényl)-5-hydroxy-3-méthyl-1H-pyrazol-4-yl]azo}-3-hydroxybenzoïque ;
- l'acide 5-chloro-3-{[1-(3,4-diméthylphényl)-5-hydroxy-3-méthyl-1H-pyrazol-4-yl]azo}-2-hydroxybenzènesulfonique ;
- l'acide 3-*tert*-butyl-4-{[1-(3,4-diméthylphényl)-5-hydroxy-1H-pyrazol-4-yl]azo}-3-hydroxybenzoïque ;
- l'acide 4-{[1-(3,4-diméthylphényl)-5-hydroxy-3-phényl-1H-pyrazol-4-yl]azo}-3-hydroxybenzoïque ;
- le 3-hydroxy-4-{[5-hydroxy-3-méthyl-1-(4-méthylphényl)-1H-pyrazol-4-yl]azo}benzoate de méthyle ; et
- l'acide 4-{[(1-(4-*tert*-butylphényl)-5-hydroxy-3-méthyl-1H-pyrazol-4-yl]azo}-3-hydroxybenzoïque ;
et leurs sels, hydrates, solvates et esters pharmaceutiquement acceptables.

6. Utilisation d'un composé de Formule (II) selon l'une quelconque des revendications précédentes, dans la fabrication d'un médicament,
dans laquelle le médicament est destiné à être utilisé dans l'administration à un mammifère, d'une quantité thérapeutiquement efficace du composé, et à stimuler la production de plaquettes chez le mammifère,
et dans laquelle le mammifère, humain inclus, nécessite la stimulation de la production de plaquettes.

7. Utilisation d'un composé de Formule (II) selon l'une quelconque des revendications précédentes, dans la fabrication d'un médicament destiné à être utilisé dans l'administration d'une quantité efficace du composé à un sujet et à agir comme agoniste du récepteur TPO chez ledit sujet.

8. Composition pharmaceutique pour l'utilisation dans le traitement de thrombocytopénie, qui comprend un composé de Formule (II) selon l'une quelconque des revendications précédentes, et un véhicule pharmaceutiquement acceptable.

9. Composition pharmaceutique pour l'utilisation en administration dans le but de stimuler la production de plaquettes chez un mammifère, humain inclus, nécessitant la stimulation de la production de plaquettes,
laquelle composition comprend une quantité thérapeutiquement efficace d'un composé de Formule (II) selon l'une quelconque des revendications précédentes, et un véhicule pharmaceutiquement acceptable.

10. Utilisation d'une composition selon l'une quelconque des revendications précédentes, dans laquelle le médicament ou la composition est destiné à l'administration orale.

11. Utilisation d'une composition selon l'une quelconque des revendications précédentes, dans laquelle le médicament ou la composition est destiné à l'administration parentérale.

12. Médicament à usage thérapeutique, fabriqué en utilisant, et comprenant un composé représenté par la Formule (I) suivante : 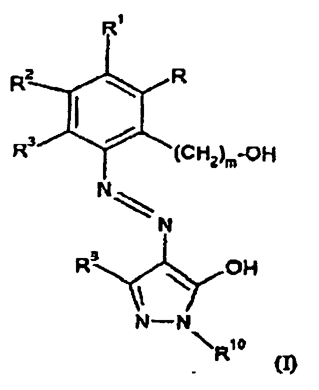
dans laquelle ;
- R, R¹, R², R³ et R⁹ sont chacun indépendamment choisis parmi l'hydrogène, les groupes alkyle C₁₋₆, alcoxy C₁₋₆, -(CH₂)ₚOR⁴, -C(O)OR⁴, nitro, cyano, halogène, aryle, -S(O)ₙR⁴, cycloalkyle, -CONR⁵R⁶, acide phosphonique, acide sulfonique, acide phosphinique et -SO₂NR^{S}R⁶;
où
- p est de 0 à 6 ;
- n est de 0 à 2 ;
- R⁴ représente l'hydrogène, un groupe alkyle, cycloalkyle, aryle C₁-C₁₂, alkyle substitué, cycloalkyle substitué et aryle C₁-C₁₂ substitué, et
- R⁵ et R⁶ sont choisis chacun indépendamment parmi l'hydrogène, les groupes alkyle, cycloalkyle C₃₋₆, aryle, ou bien R⁵ et R⁶ pris en association avec l'azote auquel ils sont rattachés, représentent un cycle à saturé de 5 à 6 atomes contenant jusqu'à un autre hétéroatome choisi parmi l'oxygène et l'azote ;
- m est de 0 à 6 ; et
- R¹⁰ représente un cycle aromatique cyclique ou polycyclique contenant 3 à 16 atomes de carbone et contenant éventuellement un ou plusieurs hétéroatomes, à la condition que lorsque le nombre d'atomes de carbone est égal à 3, le cycle aromatique contienne au moins deux hétéroatomes et que lorsque le nombre d'atomes de carbone est égal à 4, le cycle aromatique contienne au moins un hétéroatome, et éventuellement substitué par un ou plusieurs substituants choisis au sein du groupe consistant en des groupes alkyle, cycloalkyle, cycloalkyle substitué, aryle, aryle substitué, aryloxy, alcoxy, acyloxy, amino, nitro, cyano, halogène, hydroxy et alkyle substitué par un ou plusieurs substituants choisis au sein du groupe consistant en des groupes alcoxy, acyloxy, aryle, aryle substitué, cycloalkyle, cycloalkyle substitué, aryloxy, amino, nitro, cyano, halogène et hydroxy ; et ses sels, hydrates, solvates et esters pharmaceutiquement acceptables ;
et dans laquelle
par le terme « aryle », sauf mention contraire, il est désigné un cycle aromatique cyclique ou polycyclique contenant de 1 à 14 atomes de carbone et contenant éventuellement un à cinq hétéroatomes, à la condition que lorsque le nombre d'atomes de carbone est de 1, le cycle aromatique contienne au moins quatre hétéroatomes, que lorsque le nombre d'atomes de carbone est de 2, le cycle aromatique contienne au moins trois hétéroatomes, que lorsque le nombre d'atomes de carbone est de 3, le cycle aromatique contienne au moins deux hétéroatomes et que lorsque le nombre d'atomes de carbone est de 4, le cycle aromatique contienne au moins un hétéroatome ;
par le terme « aryle C₁-C₁₂ », sauf mention contraire, il est désigné les groupes phényle, naphtalène, 3,4-méthylènedioxyphényle, pyridine, biphényle, quinoléine, pyrimidine, quinazoline, thiophène, furanne, pyrrole, pyrazole, imidazole et tétrazole :
par le terme « substitué », sauf mention contraire, il est signifié que l'entité chimique concernée a un ou plusieurs substituants choisis au sein du groupe consistant en des groupes hydroxyalkyle, alcoxy, acyloxy, alkyle, amino, N-acylamino, hydroxy, -(CH₂)_{g}C(O)OR¹¹, -S(O)ₙR¹², nitro, cyano, halogène et trifluorométhyle, où g est de 0 à 6, R¹¹ représente l'hydrogène ou un groupe alkyle, n est de 0 à 2 et R¹² représente l'hydrogène ou un groupe alkyle ;
par le terme « alcoxy » il est désigné un groupe -O-alkyle dans lequel le groupe alkyle est tel que décrit ici ;
par le terme « cycloalkyle », sauf mention contraire, il est désigné une entité non aromatique C₃-C₁₂, saturée ou insaturée, cyclique ou polycyclique ;
par le terme « acyloxy », il est désigné un groupe -OC(O)alkyle dans lequel le groupe alkyle est tel que décrit ici ;
par le terme « N-acylamino », il est désigné un groupe -N(H)C(O)alkyle dans lequel le groupe alkyle est tel que décrit ici ;
par le terme « aryloxy », il est désigné un groupe -O-aryle C₆-C₁₂ dans lequel aryle C₆-C₁₂ représente le groupe phényle, naphtyle, 3,4-méthylènedioxyphényle ou biphényle éventuellement substitué par un ou plusieurs substituants choisis au sein du groupe consistant en des groupes alkyle, hydroxyalkyle, alcoxy, trifluorométhyle, acyloxy, amino, N-acylamino, hydroxy, -(CH₂)_{g}C(O)OR¹¹, -S(O)ₙR¹², nitro, cyano et halogène, où g est de 0 à 6, R¹¹ représente l'hydrogène ou un groupe alkyle, n est de 0 à 2 et R¹² représente l'hydrogène ou un groupe alkyle ;
par le terme « hétéroatome », il est désigné l'oxygène, l'azote ou le soufre ;
par le terme « halogène » tel qu'il est employé ici, il est désigné un substituant choisi parmi les groupes bromure, iodure, chlorure et fluorure ; et
par le terme « alkyle » et ses dérivés et dans toutes les chaînes carbonées, tel qu'il est employé ici, il est désigné une chaîne hydrocarbonée linéaire ou ramifiée, saturée ou insaturée, et sauf mention contraire, la chaîne carbonée contiendra de 1 à 12 atomes de carbone ;
à la condition que
au moins l'un de R, R¹, R² et R³ représente le groupe acide sulfonique, tétrazole, -CONR⁵R⁶, acide phosphonique ou acide phosphinique ; où R⁴, R⁵ et R⁶ sont tels que décrits plus haut ;
et à la condition que
lorsque R¹ représente un acide carboxylique ; R, R² et R³ représentent l'hydrogène ; et R⁹ représente le groupe méthyle ; R¹⁰ ne représente pas un groupe phényle non substitué.

13. Médicament selon la revendication 12, dans lequel, dans le composé de Formule (I)
- R¹ représente un acide carboxylique ou un acide sulfonique ;
- R, R² et R³ sont chacun choisis indépendamment parmi l'hydrogène, les groupes alcoxy C₁₋₆, tétrazole, -CONR⁵R⁶, acide phosphonique, acide phosphinique, alkyle C₁₋₆ et halogène ;
- m est égal à 0 ; et
- R⁹ représente un groupe alkyle C₁₋₆, alcoxy C₁₋₆, halogène ou aryle C₁-C₁₂; et
- R'° représente un groupe phényle substitué par un ou plusieurs substituants choisis au sein du groupe consistant en des groupes alkyle, alkyle substitué, alcoxy, trifluorométhyle, halogène et hydroxy.

14. Médicament selon la revendication 12, dans lequel, dans le composé de Formule (I)
- R¹ représente un acide carboxylique ou un acide sulfonique ;
- R, R² et R³ sont chacun choisis indépendamment parmi l'hydrogène, les groupes alcoxy C₁₋₆, alkyle C₁₋₆ et halogène ;
- m est égal à 0 ; et
- R⁹ représente un groupe alkyle C₁₋₆, ou alcoxy C₁₋₆; et
- R¹⁰ représente un groupe phényle substitué par un à trois substituants choisis au sein du groupe consistant en des groupes alkyle, hydroxy, alcoxy, trifluorométhyle et halogène.

15. Médicament selon les revendications 12, 13 ou 14, dans lequel, dans le composé de Formule (I)
le terme « alkyle » y compris ses dérivés, désigne les groupes -CH₃, -CH₂-CH₃, -CH₂-CH₂-CH₃, -CH(CH₃)₂, -C(CH₃)₃, -(CH₂)₃-CH₃, -CH₂-CH(CH₃)₂ ou -CH(CH₃)-CH₂-CH₃,
et le terme « substitué » signifie que l'entité chimique concernée a un substituant choisi au sein du groupe consistant en des groupes hydroxyalkyle, alcoxy, acyloxy, alkyle, amino, N-acylamino, hydroxy, C(O)OR¹¹, -S(O)ₙR¹², nitro, cyano, halogène et trifluorométhyle, où R¹¹ représente l'hydrogène ou un groupe alkyle, n est de 0 à 2 et R¹² représente l'hydrogène ou un groupe alkyle.

16. Médicament selon la revendication 12, dans lequel le composé de Formule (I) est choisi parmi :
- l'acide 3-hydroxy-4-{[5-hydroxy-3-méthyl-1-(4-méthylphényl)-1H-pyrazol-4-yl]azo}benzoïque ;
- l'acide 4-{[1-(4-benzyloxyphényl)-5-hydroxy-3-méthyl-1H-pyrazol-4-yl]azo}-3-hydroxybenzoïque ;
- l'acide 4-{[1-(4-chlorophényl)-5-hydroxy-3-méthyl-1H-pyrazol-4-yl]azo}-3-hydroxybenzoïque ;
- l'acide 4-{[1-(3-chlorophényl)-5-hydroxy-3-méthyl-1H-pyrazol-4-yl]azo}-3-hydroxybenzoïque ;
- l'acide 4-{[1-(3,4-diméthylphényl)-5-hydroxy-3-méthyl-1H-pyrazol-4-yl]azo}-3-hydroxybenzoïque ;
- l'acide 3-{[1-(3,4-diméthylphényl)-5-hydroxy-3-méthyl-1H-pyrazol-4-yl]azo}-4-hydroxybenzoïque ;
- l'acide 3-hydroxy-4-{[5-hydroxy-3-méthyl-1-(3-méthylphényl)-1H-pyrazol-4-yl]azo}benzoïque ;
- l'acide 3-hydroxy-4-{[5-hydroxy-3-méthyl-1-(4-trifluorométhylphényl)-1H-pyrazol-4-yl]azo}benzoïque ;
- l'acide 3-{[1-(3,4-diméthylphényl)-5-hydroxy-3-méthyl-1H-pyrazol-4-yl]azo}-2-hydroxybenzoïque ;
- l'acide 2-{[1-(3,4-diméthylphényl)-5-hydroxy-3-méthyl-1H-pyrazol-4-yl]azo}-3-hydroxybenzoïque ;
- l'acide 5-chloro-3-{[1-(3,4-diméthylphényl)-5-hydroxy-3-méthyl-1H-pyrazol-4-yl]azo}-2-hydroxybenzènesulfonique ;
- l'acide 3-*tert*-butyl-4-{[1-(3,4-diméthylphényl)-5-hydroxy-1H-pyrazol-4-yl]azo}-3-hydroxybenzoïque ;
- l'acide 4-{[1-(3,4-diméthylphényl)-5-hydroxy-3-phényl-1H-pyrazol-4-yl]azo}-3-hydroxybenzoïque ;
- le 3-hydroxy-4-{[5-hydroxy-3-méthyl-1-(4-méthylphényl)-1H-pyrazol-4-yl]azo}benzoate de méthyle ; et
- l'acide 4-{[1-(4-*tert*-butylphényl)-5-hydroxy-3-méthyl-1H-pyrazol-4-yl]azo}-3-hydroxybenzoïque ;
et leurs sels, hydrates, solvates et esters pharmaceutiquement acceptables.

17. Composé choisi parmi
- l'acide 3-hydroxy-4-{[5-hydroxy-3-méthyl-1-(4-méthylphényl)-1H-pyrazol-4-yl]azo}benzoïque ;
- l'acide 4-{[1-(4-benzyloxyphényl)-5-hydroxy-3-méthyl-1H-pyrazol-4-yl]azo}-3-hydroxybenzoïque ;
- l'acide 4-{[1-(4-chlorophényl)-5-hydroxy-3-méthyl-1H-pyrazol-4-yl]azo}-3-hydroxybenzoïque ;
- l'acide 4-{[1-(3-chlorophényl)-5-hydroxy-3-méthyl-1H-pyrazol-4-yl]azo}-3-hydroxybenzoïque ;
- l'acide 4-{[1-(3,4-diméthylphényl)-5-hydroxy-3-méthyl-1H-pyrazol-4-yl]azo}-3-hydroxybenzoïque ;
- l'acide 3-{[1-(3,4-diméthylphényl)-5-hydroxy-3-méthyl-1H-pyrazol-4-yl]azo}-4-hydroxybenzoïque ;
- l'acide 3-hydroxy-4-{[5-hydroxy-3-méthyl-1-(3-méthylphényl)-1H-pyrazol-4-yl]azo}benzoïque ;
- l'acide 3-hydroxy-4-{[5-hydroxy-3-méthyl-1-(4-trifluorométhylphényl)-1H-pyrazol-4-yl]azo}benzoïque ;
- l'acide 3-{[1-(3,4-diméthylphényl)-5-hydroxy-3-méthyl-1H-pyrazol-4-yl]azo}-2-hydroxybenzoïque ;
- l'acide 2-{[1-(3,4-diméthylphényl)-5-hydroxy-3-méthyl-1H-pyrazol-4-yl]azo}-3-hydroxybenzoïque ;
- l'acide 5-chloro-3-{[1-(3,4-diméthylphényl)-5-hydroxy-3-méthyl-1H-pyrazol-4-yl]azo}-2-hydroxybenzènesulfonique ;
- l'acide 3-*tert*-butyl-4-{[1-(3,4-diméthylphényl)-5-hydroxy-1H-pyrazol-4-yl]azo}-3-hydroxybenzoïque ;
- l'acide 4-{[1-(3,4-diméthylphényl)-5-hydroxy-3-phényl-1H-pyrazol-4-yl]azo}-3-hydroxybenzoïque ;
- le 3-hydroxy-4-{[5-hydroxy-3-méthyl-1-(4-méthylphényl)-1H-pyrazol-4-yl]azo}benzoate de méthyle ; et
- l'acide 4-{[1-(4-*tert*-butylphényl)-5-hydroxy-3-méthyl-1H-pyrazol-4-yl]azo}-3-hydroxybenzoïque ;
et leurs sels, hydrates, solvates et esters pharmaceutiquement acceptables.

18. Composition pharmaceutique comprenant un composé représenté par la Formule (I) selon l'une quelconque des revendications 12 à 16, et un véhicule pharmaceutique.

19. Composition pharmaceutique pour l'utilisation en administration dans le but de stimuler la production de plaquettes chez un mammifère, humain inclus, nécessitant la stimulation de la production de plaquettes,
laquelle composition comprend une quantité thérapeutiquement efficace d'un composé de Formule (I) selon l'une quelconque des revendications 12 à 16, et un véhicule pharmaceutiquement acceptable.
